# EUROPÄISCHE PATENTSCHRIFT

(11) **EP 0 273 958 B1**
(45) Veröffentlichungstag und Bekanntmachung des Hinweises auf die Patenterteilung: **29.12.1993**
(21) Anmeldenummer: 87904529.2
(22) Anmeldetag: 14.07.1987
(51) Int. Cl.: A61B 5/05

(54) **VORRICHTUNG ZUM FESTSTELLEN VON EIGENSCHAFTEN, VERSCHIEDENHEITEN UND VERÄNDERUNGEN DES MENSCHLICHEN ODER TIERISCHEN KÖRPERS**
DEVICE FOR DETECTING PROPERTIES, PARTICULARITIES AND CHANGES OF HUMAN OR ANIMAL BODIES
DISPOSITIF DE DETECTION DE PROPRIETES, DE PARTICULARITES ET DE MODIFICATIONS DE CORPS HUMAINS OU ANIMAUX

(30) Priorität: 14.07.1986 DE 3623711
(43) Veröffentlichungstag der Anmeldung: 13.07.1988
(73) Patentinhaber: HANDELSGESELLSCHAFT FÜR MEDIZIN UND TECHNIK MIT BESCHRÄNKTER HAFTUNG, D-85221 Dachau (DE)
(72) Erfinder: KIRCHHOFF, Günter, D-8060 Dachau (DE)
(74) Vertreter: Beszédes, Stephan G., Dr.
(86) Internationale Anmeldenummer: EP8700378
(87) Internationale Veröffentlichungsnummer: WO8800450

(56) Entgegenhaltungen:
- CH-B- 547 642
- DE-A- 1 598 910
- DE-B- 1 006 539
- DE-B- 1 126 559
- DE-C- 961 827
- DE-U- 8 520 235
- FR-A- 1 439 457
- GB-A- 342 419
- GB-A- 431 672
- US-A- 1 746 379
- US-A- 2 930 977
- US-A- 4 175 551
- US-A- 4 240 445
- US-A- 4 365 637

## Beschreibung

Die Erfindung betrifft eine Vorrichtung zur Feststellung Von Inhomogenitäten in lebenden und toten menschlichen und tierischen Körpern mittels hochfrequenter elektrischer Spannungen und Ströme geringer Größe. Gemessen wird die durch die verwendete hochfrequente Elektrizität entstandene elektromagnetische Feldstärke, bezogen auf einen Bezugspunkt (Eichpunkt), am Meßkörper. Es erfolgt also eine Relativmessung. An jeder Stelle der Körperoberfläche hat die Feldenergie einen lokalen Betrag, der davon abhängt, welchen Weg das HF-Feld im Körper genommen hat und welche Medien/Inhomogenitäten mit welchen Leitfähigkeiten/Widerständen es durchdringen mußte.

Eine Wechselstromquelle ist mit einem Pol direkt oder indirekt kapazitiv an den Untersuchungskörper angeschlossen und bewirkt an diesem eine Antennenabstrahlung.

Die Meßvorrichtung besitzt eine Empfangselektrode, die an der Körperoberfläche an den zu messenden Stellen in bestimmter Weise angesetzt wird und die abgestrahlte Feldstärke mißt. Die beiden Teile der Vorrichtung - Sendeteil und Empfängerteil - können auch getrennt voneinander konstruiert sein, um in der Handhabung bestimmte Vorteile, zum Beispiel weitgehendes Vermeiden von Kabel, zu erreichen.

Zum Zwecke der Feldstärkenmessung wird nun die Empfängerelektrode (Elektrode des Tastkörpers) mit einem Abstand zu der Körperoberfläche, die es an bestimmten Punkten zu messen gilt, angeordnet.

Bei einem solchen Meßverfahren sind Vorrichtungen erforderlich, welche bei der verwendeten hochfrequenten Elektrizität keinerlei Schäden im menschlichen Körper oder anderswo verursachen können, um insbesondere ein mehrfaches, wiederholtes Messen zuzulassen, damit beispielsweise der Fortgang einer Krankheit oder deren Heilung mit Hilfe der Meßdaten, aus denen Schlüsse gezogen werden, verfolgt werden kann. Entsprechende Nachteile anderer Diagnoseverfahren, beispielsweise der Röntgendiagnostik, sollen vermieden werden.

Das Vermeiden von Schäden hängt aber auch von der sinnvollen Dimensionierung der gewählten Frequenz, der gewählten Spannung und der gewählten Ströme ab. Liegt nämlich die anregende Frequenz im Bereich von Eigenfrequenzen des Körpers, so erfolgt Absorption. Die elektromagnetisch anregbaren Teilchen geraten in Eigenschwingungen. Dies wird zum Beispiel bei den Mikrowellen-Therapiegeräten, welche diese Schwingungen benutzen, um für Heilzwecke in den Geweben Wärme zu erzeugen, benutzt. Bei solchen Geräten muß jedoch die Dosierung der zugeführten Energie sehr vorsichtig vorgenommen werden, da sonst zum Beispiel auch Verbrennungen im Gewebe entstehen können.

Auch die bekannten Röntgenstrahlen mit ihrer Fähigkeit zur Zellzerstörung liegen gegenüber den Mikrowellengeräten in einem noch höheren Frequenzbereich.

Die im eingangs genannten Hochfrequenzdiagnostikverfahren verwendete Arbeitsfrequenz liegt jedoch in einem Bereich, bei dem die Zellen und Zellmembranen Durchlaß zeigen; der Wellenwiderstand ist reell; darüber und darunter liegt der Sperrbereich. Dort wird der Wellenwiderstand imaginär, und es findet Absorption statt, so daß in das Meßergebnis unerwünschte, also imaginäre, Fakten mit eingehen. Die beispielsweise beim menschlichen Körper über jahrelange Versuche sich gezeigten sinnvollen Frequenzgrenzen lagen bei etwa 150 kHz auf der einen Seite und 950 kHz auf der anderen Seite.

Auf diesem Gebiet der Hochfrequenzdiagnostik sind keine brauchbaren Verfahren oder Konstruktionen bekannt. Ein solches mit vielfachen Mängeln behaftetes Verfahren ist in der deutschen Patentschrift 950 402 beziehungsweise die entsprechende bekanntgemachte deutsche Patentanmeldung M 7 697 VIII d/30a (und der darauf beruhenden französischen Patentschrift 1 153 724, britischen Patentschrift 842 863, schweizerischen Patentschrift 352 781 und US-Patentschrift 2 930 977) beschrieben. Auch wird auf G. Bittner, "Über ein neues Verfahren der Elektrodiagnostik" in Elektromedizin, 6 [1961] 3, Seiten 125 bis 129, in welcher das in den genannten Patentschriften erörterte Verfahren beschrieben ist, verwiesen. Ferner wurde von Paul A. Broß in seiner Broschüre "Physikalische Grundlagen der Anthroposkopie, Vorabdruck - auszugsweise - aus Propädeutik der Hochfrequenz-Diagnostik Band I: Wissenschaftliche Grundlagen", herausgegeben von der Deutschen Gesellschaft für Hochfrequenz-Diagnostik e.V. - Anthroposkopie - München 1986, das Meßverfahren eingehend begründet. Die medizinische Grundlage ist in kurzen Zügen zu finden bei: R. Kirchhoff, "Physiologische und zytologische Grundlagen für die Anthroposkopie", in Erfahrungsheilkunde - Zeitschrift für die ärztliche Praxis - acta medica empirica, 29 [1980], 8, Seiten 662 bis 664.

Aus den vielfältigen Nachteilen der bekannten Konstruktionen sollen nur einige wesentliche angeführt werden:
Die Meßelektroden zeigten bei unterschiedlichem Auflagedruck und unterschiedlicher Stellung (beispielsweise Neigung) zur Oberfläche an der gleichen Meßstelle unterschiedliche Meßwerte.

Die beschriebene Abtastsonde (deutsche Patentschrift 950 402, Seite 5, Zeilen 20 ff), nach der "der Anschlag aus Material besteht, das auf die Messung keinen Einfluß hat", ist in der Praxis nicht realisierbar. Ein solches Material ist nicht denkbar, da jeder Stoff eine bestimmte, ihm eigene relative Dielektrizitätskonstante besitzt, welche die hochfrequente Leitfähigkeit beeinflußt. Lediglich Luft würde als "Material" keinen Einfluß auf die Messung haben, weil das den Abtastkörper umgebende "Material" ebenfalls Luft ist.

Dieses Beispiel sei nur zur Verdeutlichung herausgegriffen, daß eben das genannte bekannte Verfahren keineswegs geeignet ist, in der Praxis mit den Ansprüchen an objektive Datenerfassung eingesetzt zu werden.

Auf die Wahl des Materials für die Meßelektroden war zu wenig Wert gelegt, insbesondere die Bedeutung der relativen Dielektrizitätskonstanten des Materiales bei der hochfrequenten Messung nicht genügend berücksichtigt worden. Dadurch war eine Reproduzierbarkeit der Meßergebnisse im praktischen Gebrauch der Apparate nicht möglich.

Die Apparaturen waren vielfach noch auf dem Stand der Röhrentechnik und ermöglichten schon dadurch einige Meßvorteile nicht.

Ein weiterer Nachteil war die nicht genügend mögliche Variabilität bei den Messungen, denn die Erfassung und richtige Deutung von Inhomogenitäten (beispielsweise entzündlichen Herden einerseits oder verhärteten Geschwulsten andererseits) in unterschiedlichen Tiefen gestaltete sich schwierig oder war unmöglich.

Ferner ist in der deutschen Gebrauchsmusterschrift 85 20 235 eine Vorrichtung zum Feststellen von Eigenschaften, Verschiedenheiten und Veränderungen des menschlichen lebenden oder toten Körpers mit einer Wechselstromquelle, die mit einem Pol direkt an den Untersuchungskörper angeschlossen ist, und mit einer elektrischen Meßvorrichtung, die zwischen dem anderen Pol der Wechselstromquelle und einer über die Körperoberfläche geführten Abtastelektrode angeordnet ist, wobei an der Abtastelektrode Abstandsstücke vorgesehen sind und die Abtastelektrode im Abstand zu der über den Körper geführten Fläche der Vorrichtung angeordnet ist, bekannt. Diese bekannte Vorrichtung kann auch innerhalb eines Gehäuses eine Abschirmung zum Fernhalten von Störeinflüssen von einer Elektrode, die im Abstand von der Oberfläche eines Körpers angeordnet ist, aufweisen. Diese Abschirmung ist jedoch zwingend innerhalb des Gehäuses angeordnet und ist aus diesem auch nicht herausbewegbar. Dadurch wäre es nur schwer möglich, die zum Körper, also zur Meßspitze, hin liegenden Kanten der Abschirmung für Ableitungen zu benutzen, so daß nach wie vor Signale nicht automatisch erfaßt werden können und die Kontrolle des richtigen Aufsetzens der Vorrichtung auf den Körper nur schwerfällig mittels Augenmaßes möglich ist sowie die Vorrichtung nicht gut gleichzeitig mit hoher Empfindlichkeit und ausreichend geringer Störungsverursachung ausgeführt werden kann.

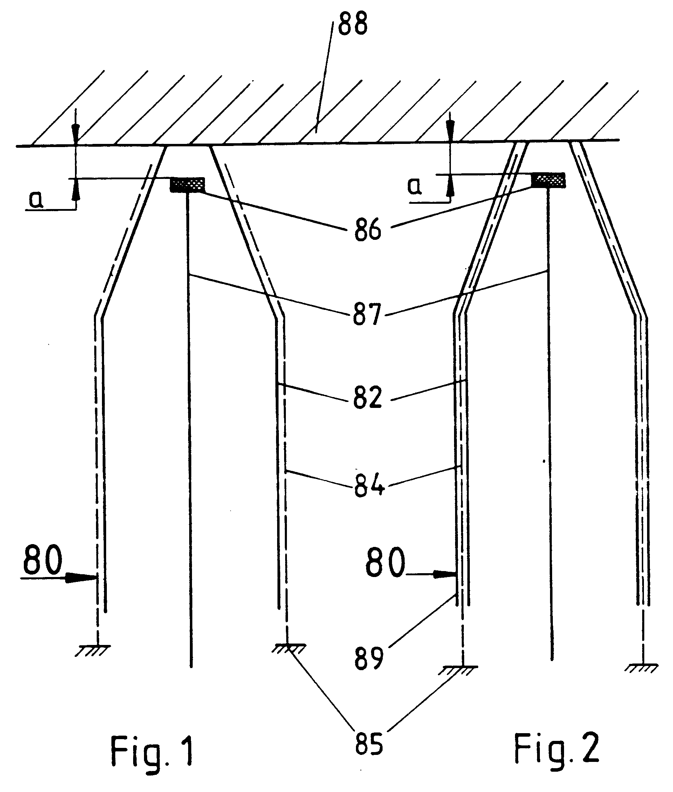

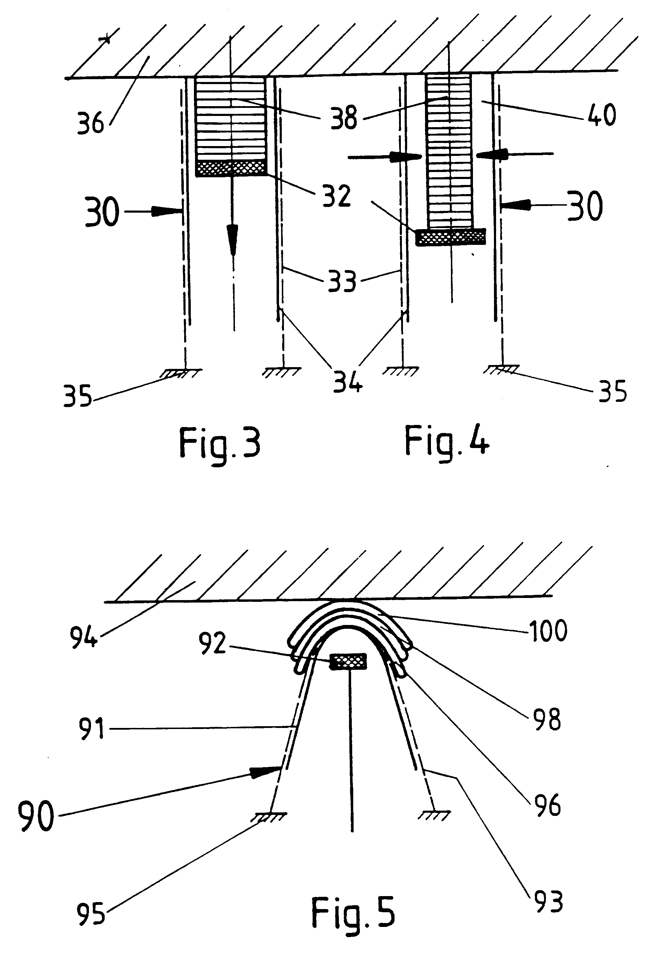

Weiterhin ist in der US-Patentschrift 4 240 445 (vergleiche insbesondere Fig. 2 und 3) eine gattungsgemäße Vorrichtung, welche auf ihrem Gehäuse eine flache Auflage mit einem Streifenleiter (Elektrode) und mit um diesen angeordneten seitlichen Abschirmungen, welche an eine ebenfalls den Streifenleiter umgebende, jedoch innerhalb des Gehäuses befindliche Abschirmung angelötet sind, sowie einer Isolierschichtauflage aufweist, beschrieben. Abgesehen davon, daß die seitlichen Abschirmungen nicht unmittelbar auf dem Gehäuse angeordnet sind, sind sie nicht mit einer Vorderkante ausgeführt, so daß eine Berührung mit dem zu messenden Körper zur Ableitung des Hochfrequenzfeldes bei dieser bekannten Vorrichtung ausgeschlossen ist. Es ist auch nicht möglich, durch unterschiedlichen Andruck dieser Vorrichtung auf die Haut die Wirkung der Abschirmung zu verändern, das heißt bei geringem Andruck zu vermindern oder bei stärkerem Andruck zu vermehren.

Weiterhin ist aus der deutschen Auslegeschrift 1 126 559 ein Gerät zum Messen durchblutungsabhängiger und pulssynchroner kapazitiver Scheinwiderstandsänderungen in Zellgeweben in der Tiefe von Hohlorganen menschlicher oder tierischer Lebewesen, wie zum Beispiel des Schädels, des Auges oder dergleichen Körperteile, mit mit einer Hochfrequenzstromquelle verbundenen, im betreffenden Objekt anzulegenden Elektroden, in welchem die pulssynchrone Änderung des biologischen Dielektrikums als Teilkapazität eines Hochfrequenzschwingkreises zu dessen Frequenzmodulation dient, welches in der Weise ausgeführt ist, daß kleinflächige Meßelektroden vorgesehen sind, zwischen denen und dem Objekt in an sich bekannter Weise ein vorzugsweise nichtleitender Körper mit insbesondere extrem großer Dielektrizitätskonstante angeordnet ist und daß dieser Körper von einem aus elastischem Werkstoff bestehenden Körper mit sehr kleiner Dielektrizitätskonstante umgeben ist und daß beide Körper gemeinsam in an sich bekannter Weise mit veränderbarem Druck gegen die Objektoberfläche anpreßbar ausgebildet sind und daß der Anpreßdruck so groß ist, daß die pulsatorischen Änderungen im blutleeren Zellgewebe unterhalb der Auflagefläche unterdrückt werden, wobei an die Elektroden zur Bestimmung der pulsatorischen Scheinwiderstandsänderungen des zwischen den Elektroden befindlichen Dielektrikums in an sich bekannter Weise eine Hochfrequenzspannung angelegt ist und der Meßkreis vorzugsweise mit einem Schwingkreis eines Oszillators zu dessen Frequenzmodulation gekoppelt ist, bekannt. Diese Druck-schrift betrifft aber nur einen Sender. In diesem Sender sind zwar eine Elektrode mit diese umgebenden seitlichen Abschirmungen vorhanden, jedoch nur im Inneren eines Gehäuses, während die Empfängerelektroden betreffend von überhaupt keiner Abschirmung die Rede ist.

Außerdem ist in der deutschen Patentschrift 961 827 eine Elektroden-Anordnung für Therapiegeräte zur medizinischen Behandlung im Kondensatorfeld kurz- beziehungsweise ultrakurzwelliger elektrischer Schwingungen, bei der die Elektrode in einer Kappe (Elektrodenschuh) mit Abstand gegen die Behandlungsfläche angeordnet ist und ein zweiter Abstand durch Vorsetzen eines Zwischenstückes (Vorschuh) vor die Kappe einstellbar ist, wobei der Mantel der Kappe leicht konisch gehalten und das Zwischenstück von geometrisch gleicher Gestalt (jedoch ohne Boden) ist, so daß sich das Zwischenstück nach dem Aufsetzen durch Keilklemmung auf der Kappe hält, beschrieben. In dieser, bei welcher es sich ebenfalls nur um einen Sender handelt, ist keine leitende Abschirmung angeordnet.

Der Erfindung liegt die Aufgabe zugrunde, unter Behebung der Nachteile der genannten besten Vorrichtungen in Weiterentwicklung derselben eine Vorrichtung zum Feststellen von Eigenschaften, Verschiedenheiten und Veränderungen des menschlichen oder tierischen lebenden oder toten Körpers mit einer Wechselstromquelle, die mit einem Pol direkt oder indirekt an den Untersuchungskörper angeschlossen ist, und mit einer elektrischen Meßvorrichtung, die zwischen dem anderen Pol der Wechselstromquelle und einem über die Körperoberfläche geführten Tastkörper mit einer Elektrode angeordnet ist, wobei an der Elektrode [ein] Abstandsstück(e) vorgesehen ist/sind und die Elektrode im Abstand zu der über den Körper geführten Fläche der Vorrichtung angeordnet ist, welche leichter und sicher handhabbar ist und weitgehend stabile Meßwerte liefert sowie auch zum automatischen Registrieren und Erfassen der Meßwerte ausführbar ist, zu schaffen.

Das Obige wurde überraschenderweise durch die Erfindung erreicht. Unabhängig von denjenigen Verbesserungen, die durch den allgemeinen Fortschritt der technischen Erkenntnisse möglich werden, sind nämlich durch die jahrelangen labormäßigen und praktischen Versuche einige hervorspringende Verbesserungen möglich geworden, insbesondere die verschiedenen Abschirmungen mit den interessanten, unerwarteten Folgen für günstiges Benutzen und reproduzierbare Meßwerte.

Gegenstand der Erfindung ist daher eine Vorrichtung zum Feststellen von Eigenschaften, Verschiedenheiten und Veränderungen des menschlichen oder tierischen lebenden oder toten Körpers mit einer Wechselstromquelle, die mit einem Pol direkt oder indirekt an den Untersuchungskörper zur Bildung eines Hochfrequenzfeldes angeschlossen ist, und mit einer elektrischen Meßvorrichtung, die zwischen dem anderen Pol der Wechselstromquelle und einem über die Körperoberfläche geführten Tastkörper mit einer Elektrode angeordnet ist, wobei an der Elektrode [ein] Abstandsstück(e) vorgesehen ist/sind und die Elektrode im Abstand zu der über den Körper geführten Fläche der Vorrichtung angeordnet ist, sowie die Elektrode und/oder das Abstandsstück und/oder das gegebenenfalls vorliegende Gehäuse mit einer oder mehreren an Masse angeschlossenen Schicht(en) beziehungsweise Abdeckung(en) [Abschirmung(en)] aus leitfähigem Material oder Anstrichmaterial, von welchen bei Vorliegen eines Gehäuses mindestens eine ganz oder teilweise außen auf dem Gehäuse oder innerhalb dessen Wand angeordnet ist, zum Abschirmen der Elektrode und/oder ihrer Leitung versehen ist, welche dadurch gekennzeichnet ist, daß die, gegebenenfalls ganz oder teilweise mit einer Isolierung versehene` Abschirmung im Meßbetrieb mit dem zu messenden Körper eine Anschlagkante mit vorgegebener Berührungslinie aufweist, über die zur Ableitung des durch die Vorrichtung erzeugten Hochfrequenzfeldes eine elektrische Verbindung mit Masse bzw. Erdpotential erfolgt, wobei die Berührungslinie am Gehäuse des Tastkörpers dadurch veränderbar ist, daß der Tastkörper zur Aufnahme von Abstandstücken verschiedener Größe eingerichtet ist, mit denen ein gewünschter Abstand zwischen der Elektrode und dem zu messenden Körper vorgegeben werden kann. "Versehen" ist hier so zu verstehen, daß die Elektrode mit der/den Abschirmung(en) nicht galvanisch leitend verbunden ist.

Die erfindungsgemäßen Abtastvorrichtungen sind meistens zylinderförmig, sie können aber auch andere Formen haben, beispielsweise kugelförmig oder prismatisch sein.

Unter Tastkörper ist der Teil des Empfängerteiles der erfindungsgemäßen Vorrichtung, welcher mit dem zu untersuchenden menschlichen oder tierischen Körper in Berührung zu bringen, in ihn einzudrücken ist, zu verstehen. Meistens ist das ein Gehäuse, muß es jedoch nicht sein, da auch Ausführungsformen ohne Gehäuse möglich sind.

Durch die an Masse oder Erdpotential, wie an niedrige, wie Masse abschirmend wirkende elektrische Spannung angeschlossene, Abschirmung wird der Einfluß der Antennenabstrahlung auf die Elektrode verändert. Die Meßanordnung wird dadurch erst gut handhabbar und weitgehend stabil in den Meßwerten.

Bei der erfindungsgemäßen Vorrichtung ist auch eine Reaktion des Körpers, zum Beispiel Wärmebildung, vermieden, wahrend sonst die sich ergebenden Meßwerte nicht den gegenwärtigen unbeeinflußten Zustand von Geweben und Organen zeigten, sondern einen bereits mit Reaktionen überfrachteten.

Durch die Anordnung mindestens einer Abschirmung außen auf dem Gehäuse und/oder innerhalb dessen Wand, welche zur Berührung mit dem zu messenden Körper und Ableitung des Hochfrequenzfeldes eingerichtet ist, werden die folgenden Vorteile erreicht.
a) Durch die außen angeordneten Abschirmungen können die zum Körper hin, also zur Meßspitze hin, liegenden Anschlagkanten der Abschirmung für Ableitungen benutzt werden, was bei innen liegenden Abschirmungen nur schwer möglich ist. Solche Ableitungen können beispielsweise zum automatischen Erfassen von Signalen (höchste Meßwerte) oder zum Anzeigen des Aufsetzwinkels des Tastkörpers benutzt werden, weil nur senkrechtes Aufsetzen ein korrektes Meßergebnis erreichen läßt.
b) Soll der Tastkörper möglichst dünn gehalten werden, um beispielsweise in den Rachenraum oder in andere nach außen offene oder geöffnete Hohlräume, wie die Vagina, gelangen zu können, muß dafür gesorgt werden, daß die Abschirmung nicht sehr nahe an der Elektrode und ihrem ableitenden Verbindungsmaterial zum Meßgerät liegt, weil dann die entstehende Kapazität den Empfang der Signale zu stark dämpft. Eine außen liegende Abschirmung ist weiter entfernt als eine innen liegende und daher günstiger.
c) In manchen staatlichen Hoheitsgebieten sind die gesetzlichen Bestimmungen für den Betrieb von Hochfrequenz-Sendern sehr streng, das heißt, daß nur eine äußerst geringe Energie für Sender mit den in Frage kommenden Arbeitsfrequenzen zugelassen ist. Daher muß durch Vermeiden einer hohen Kapazität und damit Stärkung des Empfangssignals eine möglichst hohe Empfänger-Empfindlichkeit bei möglichst geringer, das heißt der erlaubten, Sende-Energie erreicht werden. Wenn die Abschirmung weit genug von der empfangenden Elektrode und dem weiterleitenden Verbindungsmaterial, zum Beispiel Draht, entfernt ist, kann diese Bedingung erfüllt werden und man kommt mit äußerst geringen Sende-Energien aus.
d) Gelegentlich ist eine Veränderung des Wirkungsgrades der Abschirmung bei manchen Ausführungsformen von Vorteil. Eine Verschiebung der Abschirmung zur Tastspitze hin oder von ihr weg ist bei außenliegenden Abschirmungen bei manchen Ausführungsformen günstiger.

Es hat sich bei der Lösung der gestellten Aufgabe gezeigt, daß viele Vorteile - auch anfangs nicht erhoffte - durch die sehr unterschiedlich konstruierten und wirkenden Abschirmungen an den Elektroden der Tastkörper erreichbar wurden. Beispielsweise konnte die Störanfälligkeit auf ein unbedeutendes Maß gedrückt sowie die Messung mit den besonders geformten und abgeschirmten Meßelektroden automatisiert werden. Durch die jeweils eigenen Charakteristika der Elektroden mit Abschirmung ist eine Kollektion von speziell auf bestimmte Körperbereiche optimal einstellbaren Abtastkörpern bereitstellbar.

Wichtige Vorteile konnten auch erreicht werden durch die Formgestaltung und die Materialwahl des zwischen der Meßelektrode und der Körper-Meßstelle liegenden Dielektrikums, wobei als Dielektrikum jeder Stoff, gleich welchen Aggregatzustandes, prizipiell verwendbar ist. Außerdem haben die Versuche gezeigt, daß eine wahlweise Veränderung der Form, des Materiales, der Abschirmung sowie des Abstandes der Elektrode zur schnellen und besseren Differenzierung gefundener Meßergebnisse mit dem Verdacht auf Inhomogenitäten nötig sein kann, denn Form, Material, Abstand und Abschirmung verändern bei gleichem Meßort die Meßergebnisse, woraus umgekehrt auf bestimmte Eigenschaften des inhomogenen Geschehens geschlossen werden kann.

Die für den Betrieb von Hochfrequenzgeräten maßgebenden gesetzlichen Bestimmungen sind in den Staaten von unterschiedlichem Inhalt. Insbesondere sind unterschiedliche Grenzen für die elektromagnetische Störfeldgröße angegeben oder gesetzlich zu erwarten. Um Hochfrequenzgeräte dieser Art ohne größere Mühe überall betreiben zu dürfen, ist eine Standardausstattung bezüglich der Sendestärke und Modulation sowie der geräteinternen Empfängerempfindlichkeit angebracht. Diese hat aber nur dann einen Sinn, wenn mit Hilfe unterschiedlich dimensionierter und eventuell veränderbarer Empfängerelektroden (Tastelektroden) die reale Empfindlichkeit der Empfängerseite gestaltet und - was in manchen Fällen erhebliche Meßvorteile mit sich bringt - der Sender daraufhin bis an die Grenze des erlaubten Störpegels gebracht werden kann. Die erfindungsgemäße Vorrichtung mit der Konstruktion der abgeschirmten Elektroden des Tastkörpers mit vielfach variierbarer Charakteristika ist dafür geeignet.

Meßgeräte im medizinischen Einsatz erfordern auch optimale Sicherheit für Patienten und Bedienenden. Deshalb ist das Ermöglichen einer HF-Diagnose mittels unschädlicher Frequenzen und so geringer Spannungen bei trotzdem stabiler Meßwertgestaltung ein besonders großer Vorteil.

Für die praktische Handhabung des Gerätes war es auch wichtig, sowohl für die medizinische Praxis ein stationäres Gerät als auch für den eventuellen Hausbesuch ein ambulantes Gerät zu konstruieren. Durch die erwähnten Kriterien bei den Elektroden, der Stromversorgung und der Verwendung entsprechender gleicher Bauteile ergab sich auch bei Meßvorgängen am gleichen Meßkörper mit den beiden unterschiedlichen Meßgerätetypen eine totale Übereinstimmung der Meßergebnisse.

Diese wurde auch dadurch erreicht, daß bei Eichung der Empfängerelektrode alle einwirkenden Kräfte, die nicht mit der lokalen Meßstelle am menschlichen oder tierischen Patienten zu tun haben, ausgeschaltet werden. Hier sei verwiesen auf die erwähnte Literatur von Broß, insbesondere auf die Beschreibung des "passiven elektrischen Vierpols", bei dem eben nur diejenigen Feldveränderungen in den Meßwert eingehen, die an dem vierten, dem variablen Pol, nämlich an der gemessenen Stelle sich ergeben.

Vorzugsweise ist die erfindungsgemäße Vorrichtung eine solche, deren Sendeteil eine von der Abschirmung unbeeinflußt gebliebene hochfrequente Schwingung einer Frequenz von 150 bis 950 kHz ausstrahlt. Besonders bevorzugt beträgt die Frequenz 250 kHz bis 700 kHz.

Das Arbeitsgebiet dieser HF-Diagnostik liegt also vorzugsweise zwischen den beiden beim Menschen bekannten Frequenzen (150 kHz und 950 kHz), so daß innerhalb dieses Bereichs ein nahezu voller Durchlaß und keine Sperre möglich sind. Dies bedeutet, daß bei Verwendung des Meßverfahrens mit der erfindungsgemäßen Vorrichtung praktisch keine Körperreaktionen erfolgen, auch keine Umpolarisierung oder Veränderung des Hautwiderstandes an den Akupunktur- bzw. Neuralpunkten. Dadurch bleiben bei Aufsetzen der Elektrode die gesessenen Werte stabil, was bei anderen Verfahren und bekannten Geräten nicht der Fall ist.

Wenn gewünscht wird, daß die Vorderkante der Abschirmung spontan ableitet, ist die gegebenenfalls vorliegende Isolierung der Abschirmung verhältnismäßig dünn und/oder aus einem Material mit verhältnismäßig hoher Dielektrizitätskonstante ausgeführt. Wenn dagegen dies nicht gewünscht wird, ist die Isolierung verhältnismäßig dick und/oder aus einem Material mit verhältnismäßig geringer Dielektrizitätskonstante ausgeführt.

Nach einer zweckmäßigen Ausführungsform der Erfindung ragt von der beziehungsweise den Abschirmung(en) mindestens eine nach innen bewegbar aus dem Gehäuse heraus.

Nach einer anderen zweckmäßigen Ausführungsform der Erfindung ist beziehungsweise sind die Abschirmung(en) stattan Masse an Erdpotential, wie an niedrige, wie Masse abschirmend wirkende elektrische Spannung, angeschlossen.

Nach einer weiteren zweckmäßigen Ausführungsform der erfindungsgemäßen Vorrichtung ist zwischen der Elektrode und dem Ende des Gehäuses ein Freiraum vorgesehen.

Nach einer anderen zweckmäßigen Ausführungsform der erfindungsgemäßen Vorrichtung besteht das Abstandsstück aus verformbarem Material.

Nach einer weiteren zweckmäßigen Ausführungsform der erfindungsgemäßen Vorrichtung ist/sind das beziehungsweise die Abstandsstück(e), gegebenenfalls mit der Abschirmung, lösbar auf dem Gehäuse angeordnet.

Nach einer speziellen vorteilhaften Ausführungsform der erfindungsgemäßen Vorrichtung besteht/bestehen das beziehungsweise die Abstandsstück(e) mit der Abschirmung aus mehreren Materialien mit unterschiedlicher Dielektrizitätskonstante.

Gemäß einer weiteren speziellen vorteilhaften Ausführungsform der erfindungsgemäßen Vorrichtung sind zur wahlweisen Veränderung der Wirkung der Abschirmung mehrere Abstandsstücke, gegebenenfalls austauschbar oder in der Zahl veränderbar, übereinander angeordnet.

Nach einer noch weiteren speziellen vorteilhaften Ausführungsform der erfindungsgemäßen Vorrichtung ist das mit der Abschirmung versehene Teil in seiner Lage zu der Elektrode veränderbar.

Nach einer anderen zweckmäßigen Ausführungsform der erfindungsgemäßen Vorrichtung ist die Abschirmung der Elektrode lösbar zugleich mit der oder ohne die Elektrode auf dem Gehäuse angeordnet.

Bei der erfindungsgemäßen Vorrichtung kann die Abschirmung ganz oder teilweise an einem oder mehreren kugelförmigen oder scheibenförmigen Abstandsstück(en) und/oder Gehäuseteilen mit Stiel vorgesehen sein.

Nach einer noch weiteren besonders bevorzugten Ausführungsform der erfindungsgemäßen Vorrichtung ist die Abschirmung außen auf dem Gehäuse der Elektrode angebracht und ist/sind die Stärke, die Dichte und/oder die Ausdehnung der Abschirmung zur Gehäusespitze oder zur Elektrode oder zum/zu den Abstandsstück(en) hin unterschiedlich. Es ist besonders hervorzuheben, daß diese Ausführungsform die anderen Ausführungsformen mit anderen beziehungsweise spezielleren sonstigen Ausgestaltungen mit umfaßt.

Eine spezielle Ausführungsform der erfindungsgemäßen Vorrichtung hat durch eine besondere Art der Formgestaltung der Materialwahl, der Zusammensetzung des Abstandes zwischen Elektrode und Abtastspitze, durch besondere Art und Gestaltung der Abschirmung (Erdung) besonders hervorstehende Vorteile: Die praktischen Versuche - und hier hatten theoretische Überlegungen und Berechnungen keine Erfolgschancen - haben überraschenderweise ergeben, daß bei einer bestimmten Eindrückmulde an der Körperoberfläche sich die durch den Druck ergebende Gewebewölbung und daraufhin sich ergebende höhere lokale Feldstärke mit der zusätzlich wirkenden Abschirmung dergestalt kompensiert, daß der Meßwert gleich bleibt. Nicht der Druck an sich (beispielsweise in g gemessen), sondern die Folge des Drucks, die bei unterschiedlich weichem Gewebe trotzdem gleich groß gehalten werden muß, um jederzeit reproduzierbare Meßwerte zu erreichen, ist hier mit dieser Ausführungsform erreicht. Nach einer speziellen besonders vorteilhaften Ausführungsform der erfindungsgemäßen Vorrichtung ist/sind daher das Material und/oder die Größe oder Form des/der Abstandsstücke[s] und/oder des umliegenden Gehäuseraumes und/oder -teiles so auf das Material oder die Größe oder Form der Elektrode abgestimmt, daß die durch die Eindrückmulde der Meßkörperoberfläche bewirkte jeweilige Ausstrahlung der Feldstärke auf die Elektrode und/oder ihre Leitung durch die Abschirmung kompensiert wird. Bei dieser Ausführungsform ist zweckmäßig auf der Abschirmung eine Isolierung von verhältnismäßig großer Dicke und/oder aus einem Material mit verhältnismäßig geringer Dielektrizitätskonstante angeordnet. Die Kombinationswirkung von Abschirmung einerseits und Einstrahlung von Hochfrequenz auf die Elektrode andererseits ergibt sich beispielsweise dadurch, daß die metallische Schicht der Abschirmung zur Spitze des Elektrodenkörpers hin im Material dünner ausgeführt wird, gegebenenfalls mit zur Spitze hin größer werdenden Löchern versehen wird dadurch keine so starke Abschirmung auf die Elektrode bewirkt wie der materialstärkere Teil oder ungelöcherte Teil der Abschirmung. Umgekehrt wirkt bei größeren Eindruck des Tastkörpers in die Körperoberfläche die Abstrahlung der Hochfrequenz stärker, weil sie auch seitlich einwirkt. Je stärker aber der Tastkörper eingedrückt wird, um so mehr kommt wie beschrieben die stärkere Abschirmung zur Wirkung, und umgekehrt, das heißt je weniger tief der Tastkörper eingedrückt wird, desto geringer ist die Einstrahlungsstärke der Hochfrequenz und desto geringer kann die Abschirmungswirkung durch die beschriebene andere Dimensionierung des Abschirmungsmaterials sein. Bei diese Umstände berücksichtigenden Dimensionierungen von Tastkörper, Abstand von Spitze zu Elektrode, Elektrodenkörper und Abschirmung ergibt sich die Möglichkeit einer nahezu totalen Kombination der Werte, so daß sich der sich bei der Messung ergebende Meßwert auch bei unterschiedlichstem Andruck in gleicher Höhe zeigt.

Es gilt allgemein, daß zweckmäßig die Stärke und das Material der Isolierung der Abschirmung nach der gewünschten Ableitintensität der Anschlagkante an Masse oder Erdpotential ausgeführt ist. Speziell die im vorstehenden Absatz beschriebene Ausführungsform betreffend ist die Isolierung verhältnismäßig dick und/oder aus einem Material mit verhältnismäßig geringer Dielektrizitätskonstante ausgeführt, um eine spontane Ableitung an den Anschlagkanten der Abschirmung zu verhindern.

Nach einer speziellen vorteilhaften Ausführungsform der erfindungsgemäßen Vorrichtung ist außen am Gehäuse des Tastkörpers die Anschlagkante als mit einem an Masse oder Erdpotential gelegter Ring mit Abschirmung, der fest oder in seiner Stellung veränderbar ist, ausgebildet.

Vorzugsweise ist der Ring mit Abschirmwirkung entweder mit einer außen auf dem Gehäuse des Tastkörpers aufgebrachten Metallisierung oder über leitende Befestigungen oder Kontaktvorrichtungen mit der innerhalb des Gehäuses des Tastkörpers liegenden Abschirmung verbunden.

Nach einer speziellen vorteilhaften Ausführungsform der erfindungsgemäßen Vorrichtung ist der Ring mit Abschirmwirkung gegen einen solchen mit kleinerer oder größerer Außenfläche oder mit kleinerem oder größerem Rand veränderbar.

Nach einer speziellen vorteilhaften Ausführungsform der erfindungsgemäßen Vorrichtung ist die als Masseablei - tung wirksame metallische oder metallisierte Berührungslinie [Anschlagkante] am Gehäuse des Tastkörpers dadurch veränderbar, daß sie zur Aufnahme wahlweise aufzunehmender isolierender Kappen verschiedener Größe und dadurch Verschiebbarkeit der wirksamen Berührungslinie [Anschlagkante] eingerichtet ist.

Die erfindungsgemäße Vorrichtung mit Tastkörper mit besonderer Außenabschirmung bringt nun die möglichkeit einer automatischen Datenerfassung, - verarbeitung und -darstellung. Durch die Ableitung der Hochfrequenz an Masse oder Erdpotential exakt in dem Moment, in welchem jeweils eine bestimmte Eindrückmulde auf der Körperoberfläche entstanden ist, werden die Meßverhältnisse an allen Meßpunkten identisch. Auch eine Wiederholung der Messung am selben Meßpunkt ergibt den exakt gleichen Wert wie zuvor. Der Computer braucht nur jeweils den höchsten Wert zu registrieren, eventuell bei mehreren zeitlich hintereinanderfolgenden Messungen an derselben Meßstelle den Durchschnittswert dieser höchsten Werte ermitteln und dann wie üblich automatisch verarbeiten. Nachdem vorher die Anlage mit der gleichen Meßart - also Eindrücken des Tastkörpers, bis ein höchster Wert registriert ist - an einem Körpereichpunkt (Bezugspunkt) geeicht wurde, ergeben sich dann beim Messen stete reproduzierbare Meßwerte, freilich bei Vorhandensein von Inhomogenitäten in unterschiedlicher Höhe, was gerade zur Diagnosebestimmung ermittelt werden soll. Mit dieser automatisierten Messung können sich auch keine Fehler beim Ablesen des Instrumentes und beim Aufschreiben der Daten einschleichen.

Nach einer ganz besonders bevorzugten Ausführungsform der erfindungsgemäßen Vorrichtung ist sie an ein Meßdatenregistriergerät (Computer) zur stetigen automatischen Erfassung der sich beim Meßvorgang in Funktion des Andruckes ergebenden Meßdaten mit abrufbarer Speicherung derjenigen beiden Höchstwerte oder eines der beiden, welche sich unmittelbar vor dem Beginn der Berührung und unmittelbar nach der Beendigung der Berührung der Anschlagkanten mit Abschirmwirkung mit dem zu untersuchenden Körper einstellen, anschließbar.

Diese Ausführungsform arbeitet detailliert beschrieben wie folgt. Der Messende drückt an der betreffenden zu untersuchenden Körperstelle den Tastkörper mit der Empfängerelektrode immer mehr an, wodurch vom Meßdatenregistriergerät ein immer mehr zunehmender Wert erfaßt wird. Dies geht solange, bis die Anschlagkanten der Abschirmung von der zu untersuchenden Körperstelle erreicht sind (kurz davor wird ein Höchstwert erreicht), in welchem Moment der Wert sprunghaft absinkt, und er bleibt auch bei noch mehr zunehmendem Andruck auf Tiefwerten. Beim Entlasten des Andruckes durch Herausziehen des Tastkörpers bleiben solange Tiefwerte bestehen, bis die Trennung der Anschlagkanten der Abschirmung von der zu untersuchenden Körperstelle erreicht ist. In diesem Moment wird vom Meßdatenregistriergerät sprunghaft wieder ein Höchstwert erfaßt. Nur diese beiden Höchstwerte werden vom Meßdatenregistriergerät abrufbar gespeichert und nur bei Erfassen dieser beiden Höchstwerte ist ein abrufbarer Meßwert zweckmäßigerweise als Durchschnittswert zu erhalten. Wenn daher der Messende unrichtigerweise den Tastkörper nicht so weit andrückt, daß der Höchstwert unmittelbar vor der Berührung der Anschlagkanten mit der zu untersuchenden Körperstelle erreicht wird (es ist zwar ein Höchstwert vorhanden, aber ein falscher!), so ist kein abrufbarer Wert gespeichert und der Messende weiß, daß er unrichtig gearbeitet hat. In vereinfachter Form kann man bei entsprechender Meßsorgfalt auch mit nur einem Höchstwert auskommen.

Zweckmäßig kann die Elektrode aus verformbarem leitendem Material, das durch Druck seine Aufnahmefläche für die HF-Energie verbreitern oder verringern kann, wobei die Abschirmung nach innen mit einer isolierenden Schicht versehen ist, bestehen.

Nach einer zweckmäßigen Ausführungsform der erfindungsgemäßen Vorrichtung ist an der vorderen Seite oder Spitze des Gehäuses des Tastkörpers eine Druckknopfverbindung oder eine sonstige lösbare Verbindung zur Befestigung einer, vorzugsweise biegsamen, Fläche, wahlweise verschiedener Größe und Form mit oder ohne Abschirmung mit entsprechendem Verbindungsteil angeordnet.

Nach einer weiteren zweckmäßigen Ausführungsform der erfindungsgemäßen Vorrichtung weist das Gehäuse des Tastkörpers einen vorderen fest oder abnehmbar angeordneten Teil aus verformbarem, vorzugsweise biegsamem, Material mit oder ohne Abschirmung auf, das sich der Körperoberfläche angleicht.

Nach einer speziellen vorteilhaften Ausführungsform der erfindungsgemäßen Vorrichtung ist die innen in dem Gehäuse der Elektrode liegende metallene Abschirmung als festmontiertes oder in seiner Längsachse verschiebbares Rohr ausgebildet, das zur Veränderung der Abschirmungswirkung unterschiedlich große, unterschiedlich auf die Rohrfläche verteilte und unterschiedlich geformte Ausnehmungen besitzen kann.

Nach einer anderen speziellen vorteilhaften Ausführungsform der erfindungsgemäßen Vorrichtung weist das vordere Teil des Gehäuses des Tastkörpers eine innen angesetzte und flexibel an die Meßeinrichtung anschließbare Elektrode auf und ist verschiebbar oder verschraubbar angeordnet.

Nach einer weiteren speziellen vorteilhaften Ausfürungsform der erfindungsgemäßen Vorrichtung ist ein für Körperhohlräume verwendbarer Tastkörper mit Abschirmung in einem ein- oder mehrfach schwenkbaren Gehäuse mit ein- oder mehrfach schwenkbaren Zuleitungen angeordnet. Diese ist besonders zur Testung von Zahnfleisch oder anderen Stellen des Rachengebietes geeignet, auch für die Vagina.

Nach einer noch weiteren speziellen vorteilhaften Ausführungsform der erfindungsgemäßen Vorrichtung besteht ein Vorderteil des Gehäuses des Tastkörpers aus einer ein- und ausdrehbaren Verstellschraube zur Veränderung des Abstandes zwischen Elektrode und Meßoberfläche und zur Veränderung der Wirkung der Abschirmung.

Dabei ist es bevorzugt, daß die Verstellschraube einen verformbaren unteren Teil zum Füllen des sich beim Herausdrehen der Verstellschraube bildenden Zwischenraumes mit elastischem verformbarem Material aufweist, das sich beim Hineindrehen zusammendrückt.

Die erwähnten Besonderheiten bei der Dimensionierung der Frequenz, der Spannung, des Stromes sowie des an den Patienten anzulegenden abgeschirmten Tastkörpers der erfindungsgemäßen Vorrichtung machen eine zusätzliche Diagnostik möglich:

Die bekannten Meßgeräte der Akupunktur erreichen nur sehr schwer einen stabilen Meßwert an den Akupunktur- oder Neuralpunkten. Wenn in der erfindungsgemäßen Vorrichtung der Tastkörper als metallene Spitze oder metallener Stift mit sehr geringem Abstand zur Haut ausgebildet wird, dann können die Akupunktur- und Neuralpunkte einwandfrei aufgespürt werden.

Der geringe Abstand kann beispielsweise durch einfache Lackierung oder anderweitigen dünnen Auftrag erreicht werden. Zugleich ist die Abschirmung der Elektrode deswegen sinnvoll, damit nicht seitliche Einflüsse das Meßergebnis stören. Die Abschirmung enthält unterhalb zur Elektrode hin eine Isolierschicht und kann auch außen einen Überzug besitzen. Mit einer Kante an der Spitze des Tastkörpers kann die gefundene Körperstelle, beispielsweise der Akupunkturpunkt, markiert werden, um kurz danach exakt an dieser Stelle therapieren zu können, beispielsweise mit einer Nadel.

Nach einer besonderen bevorzugten speziellen vorteilhaften Ausführungsform der erfindungsgemäßen Vorrichtung besteht daher das Abstandsstück der Elektrode allein aus einer Lackierung oder einer anderen dünnen Auflage, welche mehr oder weniger ausgedehnt mit einer Abschirmung versehen sein kann, die über die ebenfalls lackierte oder eine andere dünne isolierende Auflage aufweisende Elektrode sich erstrecken kann.

Nach einer weiteren zweckmäßigen Ausführungsform der erfindungsgemäßen Vorrichtung weist die isolierte Elektrode an ihrer vorderen Spitze eine beliebig geformte Kante zum leichten Markieren einer gefundenen Meßstelle auf der Haut auf.

Die erfindungsgemäße Vorrichtung ermöglicht also eine zuverlässige und unbeeinflußte Punktsuche, was auch für die nicht ärztliche Handhabung im Rahmen der sogenannten Selbsttherapie von Bedeutung ist, weil über bestimmte bekannte Akupunktur- und Neuralpunkte eine kurzfristige und für eine angemessene Zeit wirksame Schmerztherapie möglich wird. Falls dieses Anwendungsgebiet für den Privatgebrauch isoliert verfolgt wird, werden auch noch Vereinfachungen beim Sendeteil und beim Empfängerteil der Anlage möglich, damit mit noch viel geringeren Spannungen gearbeitet werden und die Vorrichtung in einem sehr kleinen Gehäuse und mit angesetzter Tastspitze konstruiert werden kann. Dabei ist keinerlei Kabel nötig, da der erforderliche Kontakt im Kreislauf einerseits über die Handberührung des Gerätes (Metallkontaktplatte), andererseits über die Berührung des Patienten durch die behandelnde Person erfolgt, wenn Fremdbehandlung vorgenommen wird.

Nach einer zweckmäßigen Ausführungsform der erfindungsgemäßen Vorrichtung sind daher Sende- und Empfängerteil mit Stromversorgung in einem gemeinsamen Gehäuse untergebracht, keine Verbindungskabel von Sendeteil zum zu untersuchenden Körper und keine Verbindungskabel vom Empfängerteil zum Tastkörper vorhanden und es ist am Gerät ein metallenes Kontaktteil für das Herstellen des erforderlichen elektrischen Senderkontaktes über Handberührung und dadurch Leiten der Sendeenergie durch den zu untersuchenden Körper, gegebenenfalls unter Zwischenschaltung des Körpers der behandelnden Person, angeordnet. Bei dieser Ausführungsform erfolgt also bei Fremdbehandlung die Energieweitergabe über die Handberührung der behandelnden Person an den zu untersuchenden Körper, während bei Selbstbehandlung die Zwischenschaltung der behandelnden Person entfällt, indem dieser das metallene Kontaktteil selbst berührt und damit die Senderenergie unmittelbar weiterleitet.

Das Ziel, technische Konstruktionen zu vereinfachen und beispielsweise Kabel einzusparen oder wenigstens in ihrer Länge zu reduzieren, liegt auf der Hand und wäre an sich nicht erwähnenswert. Bei der erfindungsgemäßen Vorrichtung bilden aber Hochfrequenzkabel mit Abschirmung (Koaxialkabel) eine Meßfehlerquelle, da die äußerst geringen Spannungen und Ströme schon bei sehr geringen Kapazitätsänderungen variieren können, wenn sich zum Beispiel durch Biegen des Kabels die Kapazität zwischen Seele und Abschirmung verändert.

Während bei den Messungen am Menschen diese Fehlerquellen durch entsprechende Kabelwahl, sorgfaltiges Bewegen und Nacheichen ausgeschaltet werden können, haben Konstruktionen ohre oder mit wenig Kabel für die tiermedizinische Diagnostik eine größere Bedeutung, weil hier oft längere Kabel verwendet werden müssen.

So ist nach einer zweckmäßigen Ausführungsform der erfindungsgemäßen Vorrichtung, insbesondere zur Tiertestung, das Gehäuse des Tastkörpers mit der Elektrode und mehr oder weniger starker und unterschiedlich angebrachter Abschirmung fest oder schwenkbar oder auswechselbar am Gehäuse, welches das Sende- und das Empfängerteil ohne Verbindungskabel zum Tastkörper aufweist, angeordnet.

Bei dieser Ausführungsform weist die mit Kabel verbundene Sendeelektrode eine mit Spitzen, Höckern oder anderen Erhebungen versehene Auflagefläche auf.

Zweckmäßig weist die Sendeelektrode eine Vorrichtung zum Befestigen an dem Körper, wie Riemen oder Schnüre durch Löcher, Klettverschlüsse oder andere Befestigungselemente, auf.

Vorteile werden auch durch die Trennung von Sendeteil und Empfängerteil erreicht, weil die gegenseitige Beeinflußbarkeit, wie sie in einem gemeinsamen Gehäuse besteht, vollkommen aufgehoben wird. Dadurch sind Konstruktionselemente einsetzbar, die sonst nur mit Mühe von anderen abgeschirmt werden können. Auch sind dadurch zwei Stromversorgungsmöglichkeiten, zum Beispiel Akkumulatoren, einsetzbar, wobei jede einzelne gering dimensioniert sein und somit auf handelsübliche Produkte zurückgegriffen werden kann. Natürlich ist auch eine zentrale Stromversorgung möglich.

Es ist auch bevorzugt, daß das Sendeteil an einer oder mehreren Seiten mit metallenen oder sonstigen leitenden nach außen gerichteten Teilen, gegebenenfalls mit Höckern oder sonstigen Erhebungen, zur Kontakgabe mit der Tierhaut ausgebildet ist.

Für die Tiertestung ist es zweckmäßig, den Sendeteil der Anlage weitgehend zu kapseln, das heißt schmutz- und feuchtigkeitssicher zu gestalten, um ihn auch besser säubern zu können, was schon wegen des Gebrauchs des Kontaktgels erforderlich ist.

Sofern ein getrennter Sendeteil bei der Humanmedizin eingesetzt wird, kann dieser beispielsweise durch ein Band mit Klettverschluß an einer geeigneten Körperstelle angebracht werden.

Bei gelegentlich zweckmäßiger konstruktiver Trennung vom Sendeteil und Empfängerteil der Vorrichtung haben diese jedoch eine gemeinsame Masse (Massepotential), auch zusammen mit der Abschirmung der verwendeten Elektroden. Erst dadurch wird bei getrennten Sende- und Empfängerteilen eine Meßstabilität erreicht. Nach einer zweckmäßigen Ausführungsform der erfindungsgemäßen Vorrichtung sind daher das HF-Sendeteil und das HF-Empfängerteil in getrennten Gehäusen angeordnet und sie besitzen durch Verbindung der beiden Masseteile dasselbe Massepontial wie die Abschirmung an den Tastkörpern.

Nach einer Zweckmäßigen Ausführungsform der erfindungsgemäßen Vorrichtung ist das Empfängerteil mit einem festen oder beweglichen, vorzugsweise drehbaren, Tastkörper, vorzugsweise mit einem Gehäuse für die mehr oder weniger abgeschirmte Elektrode ausgebildet.

Nach einer anderen zweckmäßigen Ausführungsform der erfindungsgemäßen Vorrichtung ist die mehr oder weniger abgeschirmte Elektrode, vorzugsweise mit einem Gehäuse, dem HF-Empfängerteil unter Zwischenschaltung eines Kabels zugeordnet.

Eine besondere Ausgestaltung der Erfindung von Bedeutung ergibt sich durch eine neue Art von Modulation der Hochfrequenz. Der einfache Ersatz der Röhren durch Transistoren brachte den hier im folgenden beschriebenen Effekt noch nicht. Erst die Verwendung von Feld-Effekt-Transistoren, gesteuert über Gate als veränderbarer Widerstand, ermöglicht eine Modulation bis 100% ohne Oberwellenbildung. Dadurch entsteht nur ein sehr geringes Eigenrauschen, und es besteht keine Kreuz-Modulations-Fähigkeit. Dadurch wird das früher häufig erforderliche Nacheichen der Vorrichtung am relativen Körperbezugspunkt kaum mehr nötig. Nach einer speziellen vorteilhaften Ausführungsform der erfindungsgemäßen Vorrichtung sind daher Feldeffekttransistoren, gesteuert über Gate als veränderbarer Widerstand, zur Modulation der Hochfrequenz im Senderteil der Vorrichtung angeordnet.

Nach einer weiteren vorteilhaften Ausführungsform der erfindungsgemäßen Vorrichtung sind über der Abschirmung isoliert und gegeneinander und/oder nach außen isoliert leitende Kontakte, welche an geringe elektrische Spannungen anschließbar sind, fest oder verschiebbar angeordnet.

Für die Isolation der leitenden Kontakte gilt das für die Isolation der Abschirmung Gesagte mit allen zweckmäßigen Ausführungsformen sinngemäß.

Gemäß einer Variante dieser Ausführungsform sind die leitenden Kontakte mit nach vorne zum zu messenden Körper gerichteten Anschlagkanten zur Berührung mit dem zu untersuchenden Körper eingerichtet.

Die Abschirmung kann abschaltbar ausgeführt sein.

Vorzugsweise sind die leitenden Kontakte an ein Registriergerät oder eine Registriervorrichtung zur automatischen Erfassung der Ansatzstellung der Vorrichtung anschließbar. An die leitenden Kontakte, beispielsweise leitenden Streifen wird jeweils eine geringe, zweckmäßig untereinander unterschiedliche, elektrische Spannung angelegt. Die Ableitungen dieser, zweckmäßig rund um den Tastkörperkopf angeordneten Kontakte gehen in einer bestimmten Reihenfolge (im Uhrzeigersinn oder ihm entgegengesetzt) an das Registriergerät, insbesondere eine Datenerfassungsanlage (Computer). Zweckmäßig ist der Tastkörper an einer Stelle markiert, um ihn stets zum Beispiel mit der Markierung nach oben gerichtet anzusetzen. Wenn nun eine falsche, gekippte Ansatzstellung des Tastkörpers auf dem zu untersuchenden Körper erfolgt, dann werden nur einige der Kontakte vom zu untersuchenden Körper berührt, wodurch die Datenerfassungsanlage nur diese registriert und beispielsweise auf dem Bildschirm anzeigt. Auch daan, wenn die leitenden Kontakte untereinander gleiche Spannung haben, kann nicht nur erkannt werden, ob die Ansatzstellung richtig oder falsch ist, sondern auf Grund der definierten Reihenfolge der Kontakte bei falscher Ansatzstellung des Tastkörpers darüber hinaus erkannt werden, nach welcher Seite hin er fälschlich gekippt ist. Das letztere wird, im Falle daß die leitenden Kontakte untereinander unterschiedliche Spannungen aufweisen, auch auf Grund dieser Eigenschaft erkannt. Der Bedienende sieht also sofort, daß nicht alle rund um den Tastkörperkopf angeordneten Kontakte ihre Daten abgeben und verbessert daraufhin seine Ansatzstellung, bis alle Kontakte ihre Daten abgeben, was angezeigt wird; der Tastkörper ist nun senkrecht aufgesetzt. Die Datenerfassung und -registrierung in der Datenerfassungsanlage kann so eingerichtet sein, daß nur bei senkrechtem Aufsetzen des Tastkörpers, also bei Berührung aller Kontakte, die Meßdaten für die Diagnose registriert werden, was so zu einer automatischen Sperre falscher Daten führt. Natürlich ist diese Ausführungsformvariante der erfindungsgemäßen Vorrichtung auch ohne Einsatz einer Datenerfassungsanlage anwendbar. Die übermittelten Daten werden dann beispielsweise an Leuchtdioden abgegeben, die ringförmig auf der Gerätefrontplatte angeordnet sein können, woraus durch die Aktivität oder Inaktivität der Dioden zu ersehen ist, ob der Tastkörper senkrecht aufgesetzt ist. Wenn beispielsweise alle Leuchtdioden aufleuchten - oder bei anderer Schaltung alle Leuchtdioden inaktiv sind -, dann ist dies ein Zeichen für senkrechtes Ansetzen. Wenn jedoch nur einige Leuchtdioden aufleuchten, dann ist dies ein Zeichen für gekipptes, falsches Ansetzen mit sich ergebenden falschen Meßwerten für die Diagnose.

Bei der vorstehend beschriebenen Ausführungsformvariante der erfindungsgemäßen Vorrichtung sind die leitenden Kontakte unmittelbar nach Anschlagkante der Abschirmung (von der Spitze des Tastkörper-Kopfes weg betrachtet) auf der Außenfläche des Tastkörpers angebracht. Zwischen der Abschirmung und den leitenden Kontakten befindet sich eine Isolierschicht, die aber auch Luft sein kann. Auch sind sie gegeneinander isoliert.

Nach einer anderen Variante der vorstehend angegebenen Ausführungsform der erfindungsgemäßen Vorrichtung sind die leitenden Kontakte zugleich als Abschirmung eingerichtet. In diesem Falle Können die geringen Spannungen, beispielsweise 2, 4, 6, 8 beziehungsweise 10 Volt, dieser leitenden Kontakte mit Abschirmfunktion wie Masse wirken. Sehr schmale Abstände zwischen den Kontakten, beispielsweise als Kontaktstreifen ausgebildet, verhindern weitgehend eine Einstrahlung auf die empfangende Elektrode. Dann wird durch die metallische beziehungsweise metallisierte Kante oder sonstige Grenze der leitenden Kontakte die Hochfrequenz bei entsprechendem Andruck abgeleitet (Spontanableitung) und zugleich durch die Berührung wie beschrieben angezeigt, ob senkrecht aufgesetzt wurde.

Nach einer noch weiteren Variante der vorstehend angegebenen Ausführungsform der erfindungsgemäßen Vorrichtung Kann sie beispielsweise bei innerhalb des Gehäuses des Tastkörpers liegenden, nach außen verschiebbaren Abschirmungen zur Definierung des Andruckes dienen, wenn beispielsweise der vordere Teil des Kopfes des Tastkörpers oder die außen liegenden leitenden Kontakte verstellbar sind und je nach Körpergebiet und Gewebehärte die Eindrückmulde auf dem zu untersuchenden Körper unterschiedlich tief gewünscht wird. Wenn der Abstand eingestellt ist und die bestimmte gewollte Eindrückmulde erreicht ist, wird dies vom Registriergerät angezeigt, beispielsweise leuchten die Dioden auf. Dann kann der Meßwert abgelesen werden. Alle Messungen mit der gleich tiefen Eindrückmulde sind dann reproduzierbar und haben - zu gleicher Zeit und am gleichen Meßort - identische Meßwerte.

Nach einer noch weiteren Variante der vorstehend angegebenen Ausführungsform der erfindungsgemäßen Vorrichtung sind die leitenden Kontakte außen auf dem Gehäuse des Testkörpers oder in seiner Wand liegend, selbst als, gegebenenfalls alleinige, Abschirmung eingerichtet. Dabei können die leitenden Kontakte innerhalb des Gehäuses verschiebbar angeordnet sein und lediglich mit ihren vorderen Teilen wahlweise aus Schlitzen aus dem Tastkörper herausgeschoben werden oder verdeckt innen bleiben. Eine mehrfache Variation der Anwendung ist möglich: Die leitenden Kontakte können im verdeckten Zustand, also zurückgezogen, lediglich als Abschirmung wirken; im mehr oder weniger herausgeschobenen Zustand können die Anschlagkanten der leitenden Kontakte zusätzlich zur Ableitung der Hochfrequenz und damit zur Anzeige des Andrucks und zur automatischen Registrierung der Höchstwerte dienen; wenn die Kontaktstreifen nur in bestimmter Reihenfolge an eine Anzeige- oder Registriervorrichtung nicht abschirmwirksam angeschlossen sind, dann dienen sie nur zur Anzeige der Ansatzstellung der Abtastvorrichtung (gekippt - falsch - oder senkrecht aufgesetzt - - richtig). Eine Kombination der beiden letztgenannten Anwendemöglichkeiten ist ebenfalls möglich.

Die Registrierung der Meßwerte kann außer mit einem Zeigerinstrument auch in anderer Weise, beispielsweise mit einer Digitalanzeige oder, wie bereits gesagt, einem Meßdatenregistriergerät (Computer) erfolgen.

Die medizinische Diagnostik befaßt sich auch mit der Feststellung der Wirkungen verabreichter Medikamente. Sofern die Medikamente an ganz bestimmten lokal umrissenen Gebieten oder Organen ihre Wirkung zeigen, ergeben sich in vielen Fällen auch veränderte Meßwerte gegenüber dem nicht therapierten Zustand. Es ergeben sich auch veränderte Meßwerte im Fortgang der Therapie, so daß man aus der Veränderung der Meßwerte auf die Norm hin auf eine Besserung und bei Veränderungen von der Norm weg auf relative Wirkungslosigkeit der Medikamente schließen kann.

Zwar ist der "passive elektrische Vierpol" wissenschaftlich bekannt und sind technische Ausführungsformen desselben in ungezählten elektrotechnischen Anlagen enthalten, doch gewinnt er in der medizinischen Diagnostik, in welcher er bisher nicht vorgefunden wird, durch die erwähnten speziellen Gegebenheiten und Erfordernisse eine große Bedeutung mit der erfindungsgemäßen Vorrichtung. Eben nur die an der konkreten Meßstelle relativ zum Eichpunkt sich ergebenden Veränderungen werden erfaßt, und das sind die gesuchten Inhomogenitäten.

Bei Kombination von zweckmäßigen Ausführungsformen der erfindungsgemäßen Vorrichtung können die Vorteile kumuliert und dadurch besonders hervorragende Ergebnisse erzielt werden

Ausführungsbeispiele der Erfindung werden nachstehend an Hand der Zeichnungen näher erläutert. Diese sind so zu verstehen, daß bei jedem Ausführungsbeispiel, auch soweit keine Isolierung auf der beziehungsweise den Abschirmung(en) eingezeichnet ist, wahlweise [eine] solche vorliegen kann beziehungsweise können. Ferner kann in jedem Ausführungsbeispiel wahlweise die beziehungsweise eine Abschirmung innerhalb der Gehäusewand angeordnet sein (nicht gezeichnet). Weiterhin kann beziehungsweise können in jedem Ausführungsbeispiel die Abschirmung(en) aus leitfähigem Material auch ohne Gehäuse vorliegen.

Soweit nichts anderes angegeben ist, werden die Abtastvorrichtungen in den Figuren im Längsschnitt gezeigt.

Fig. 1 zeigt eine Abtastvorrichtung 80 zum Feststellen von Eigenschaften eines Körpers mit einem Gehäuse 82 und einer in dem Gehäuse angeordneten Elektrode 86. Die Elektrode 86 ist über eine Leitung 87 mit einer nicht dargestellten Meßanlage verbunden. Auch bei den Ausführungsformen der anderen Zeichnungen bedeutet diese Leitung dasselbe, ohne mit einem Bezugszeichen versehen zu sein. Mit dem Ende des Gehäuses 82 ist die Vorrichtung 80 auf einen Körper 88 aufgesetzt. Der in unterschiedlicher Größe vorsehbare Abstand zwischen der Oberfläche des Körpers 88 und der Elektrode 86 ist mit a bezeichnet Seitlich auf dem Gehäuse 82 ist eine Abschirmung 84 mit Masseanschluß 85 angeordnet.

Fig. 2 zeigt die in Fig. 1 dargestellte Ausführungsform mit der einzigen Abwandlung, daß auf der Abschirmung 84 eine Isolierung 89 angeordnet ist.

Fig. 3 zeigt eine Abtastvorrichtung 30 mit einer Elektrode 32, die in einem Gehäuse 34 angeordnet ist. Die Stirnfläche des Gehäuses 34 ist auf einen Körper 36 aufgesetzt. Der Raum zwischen der Oberfläche des Körpers 36 und der Elektrode 32 ist mit einem verformbaren Material 38 aufgefüllt, so daß durch seitlichen Druck der Abstand der Elektrode 32 von der Oberfläche des Körpers 36 verändert werden kann. Beispielsweise kann in den Raum zwischen dem Material 38 und dem Gehäuse 34 Luft eintreten. Seitlich auf dem Gehäuse 34 ist wiederum eine Abschirmung 33 mit Masseanschluß 35 angeordnet.

Fig. 4 zeigt die Abtastvorrichtung 30 nach Fig. 3 mit zusammengedrücktem Material 38. Durch Vergrößerung des Zwischenraumes 40 zwischen dem verformbaren Material 38 und der Wandung des Gehäuses 34 wurde die Elektrode 32 nach innen in das Gehäuse 34 hinein verschoben, so daß der Abstand zwischen Elektrode 32 und der Oberfläche des Körpers 36 vergrößert wurde.

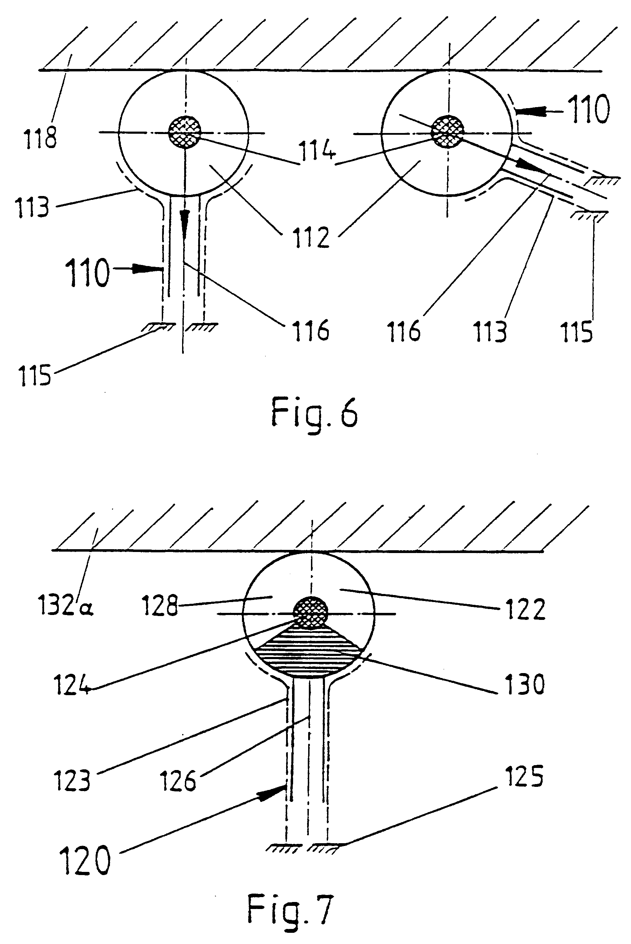

Fig. 5 zeigt eine Abtastvorrichtung 90 mit einer Elektrode 92 in einem Gehäuse 91. Zur Veränderung des Abstandes der Elektrode 92 von der zu untersuchenden Oberfläche des Körpers 94 können schalenförmige Abstandsstücke 96, 98, 100 angeordnet sein, die mit einfachen Mitteln übereinander gesteckt werden und durch ihr Vorliegen die Wirkung einer auf dem Gehäuse 91 seitlich angeordneten Abschirmung 93 mit Masseanschluß 95 wahlweise verändern können.

Fig. 6 und 7 zeigen Abtastvorrichtungen 110 bzw. 120, die einen kugelförmigen Körper 112 bzw. 122 aus einem Material mit geeigneter Dielektrizitätskonstante aufweisen. Innerhalb der Kugel 112 ist eine Elektrode 114 und innerhalb der Kugel 122 eine Elektrode 124 angeordnet. Zur Führung der Vorrichtung 110 bzw. 120 weist sie einen Stiel 116 bzw. 125 auf. Während bei der Vorrichtung 110 die Kugel 112 durchgehend aus demselben Material gefertigt ist, ist die Kugel 122 der Vorrichtung 120 aus zwei Materialien aufgebaut, nämlich einem Material 128 und einem in einem Sektor angeordneten Material 130. Die Materialien können unterschiedliche Dielektrizitätskonstanten aufweisen. Durch die kugelförmige Ausbildung des die Elektrode 114 bzw. 124 ummantelnden Körpers 112 bzw. 122 ist gewährleistet, daß bei der Führung über einen Körper 118 bzw. 132a auch beim Kippen oder Drehen immer derselbe Abstand der Elektrode 114 bzw. 124 zu dem Körper gewährleistet ist. Am Umfang der Kugel 112 bzw. 122 sowie an der Außenseite des Stiels 116 bzw. 126 ist eine Abschirmung 113 bzw. 123 mit Masseanschluß 115 bzw. 125 angeordnet.

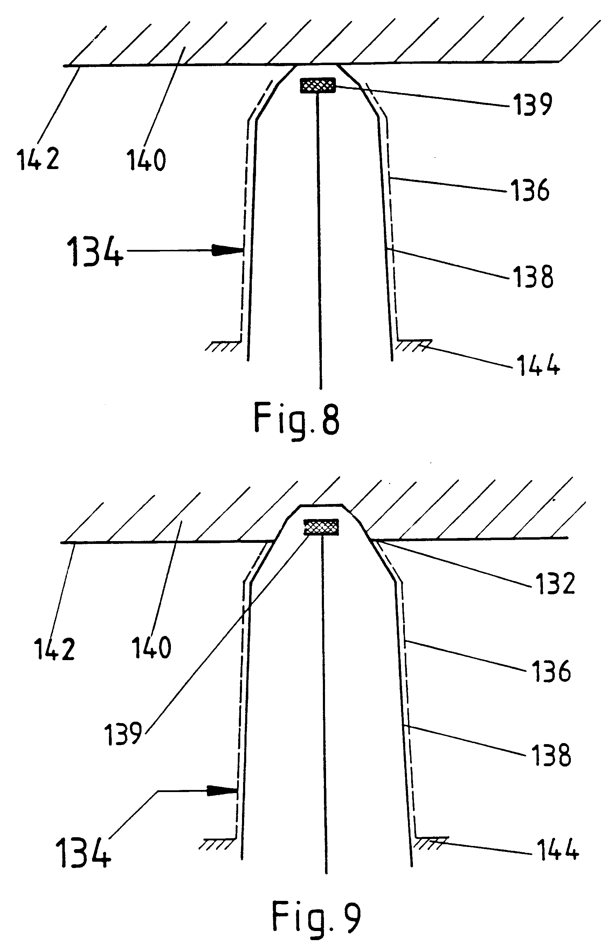

Fig. 8 zeigt in schematischer Darstellung, als weitere Ausführungsform eine Abtastvorrichtung 134 mit einer in einem Gehäuse 138 angeordneten Elektrode 139, bei welcher eine metallische Abschirmung 136, beispielsweise aufgebracht mit Silberleitlack, und angeschlossen an Masse 144 außen am Gehäuse 138 liegt, so daß eine durch starken Druck auf eine weiche Körperoberfläche 140 bewirkte Berührung der Körperhaut 142 mit der Abschirmung 136 ein rapides Absinken des Meßwertes am Meßinstrument nach sich zieht, weil das Hochfrequenzfeld an Masse 144 abgeleitet wird. Das Absinken des Meßwertes zeigt an, daß zu stark gedrückt wurde und nicht gleiche Bedingungen wie am vorher angedrückten Eichpunkt (hier nicht gezeichnet) vorliegen, wo vorher die Abtastvorrichtung so weit eingedrückt wurde, daß der höchste Meßwert erreicht wurde, also die Abschirmung gerade noch nicht berührt wurde.

Fig. 9 zeigt die in Fig. 8 dargestellte Vorrichtung, wobei die Körperhaut 142 von der Abschirmung 136 bei 132 berührt wird.

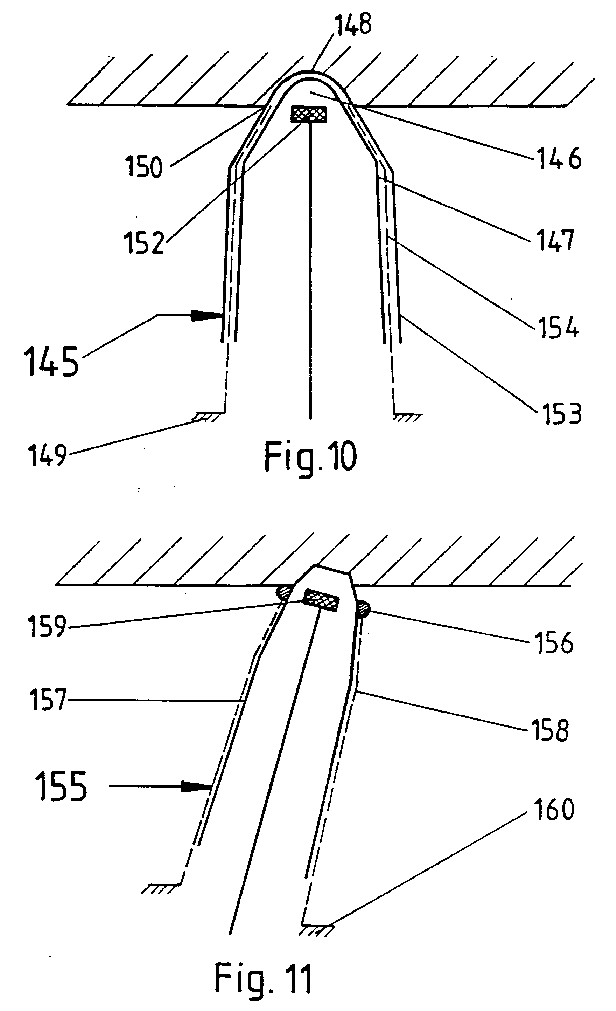

Fig. 10 stellt als weitere Ausführungsform eine Abtastvorrichtung 145 mit dem durch Versuche ermittelten, durch entsprechende Auswahl des Materiales des Gehäuses bzw. Tastkörpers 147, der Form dieses Gehäuses 147, des Materiales und der Form für den Abstand 146 zwischen Elektrode 152 und Hautaufsetzpunkt 148 sowie durch entsprechende Wahl der Materialstärke und Dichte des aufgetragenen leitfähigen Materiales der außen auf dem Gehäuse 144 angeordneten Abschirmung 154 mit Masseanschluß 149-ermöglichten Fall mit einer auf der Abschirmung 154 angebrachten Isolierung 153 mit zur Spitze zu zunehmender oder abnehmender Stärke von solcher Größe, daß die vordere Kante der unter der Isolierung 153 liegenden Abschirmung 154 nicht zu einer spontanen Ableitung der Hochfrequenz führt, dar, bei welchem die durch die Eindrückmulde auf der Körperhaut 150 bewirkte Ausstrahlung der Feldstärke auf die Elektrode 152 derart kompensiert wird, daß die Wirkung der Abschirmung 154 zu nahezu gleichen Meßwerten auf dem Anzeigeninstrument führt. Die Abschirmung 154 kann auch beispielsweise am vorderen Teil des Gehäuses 147 außen aufgetragen uni isoliert sein, dann aber innerhalb des Gehäuses 147 oder innen im Gehäuse 147 weitergeführt sein. Sämtliche Materialien und Formen sowie der Abstand zwischen Elektrode 152 und Körperaufsetzpunkt 148 müssen nach ihrer speziellen Eignung gewählt werden (auch wegen ihrer unterschiedlichen Dielektrizitätskonstanten), um diese Kompensationswirkung zu erreichen.

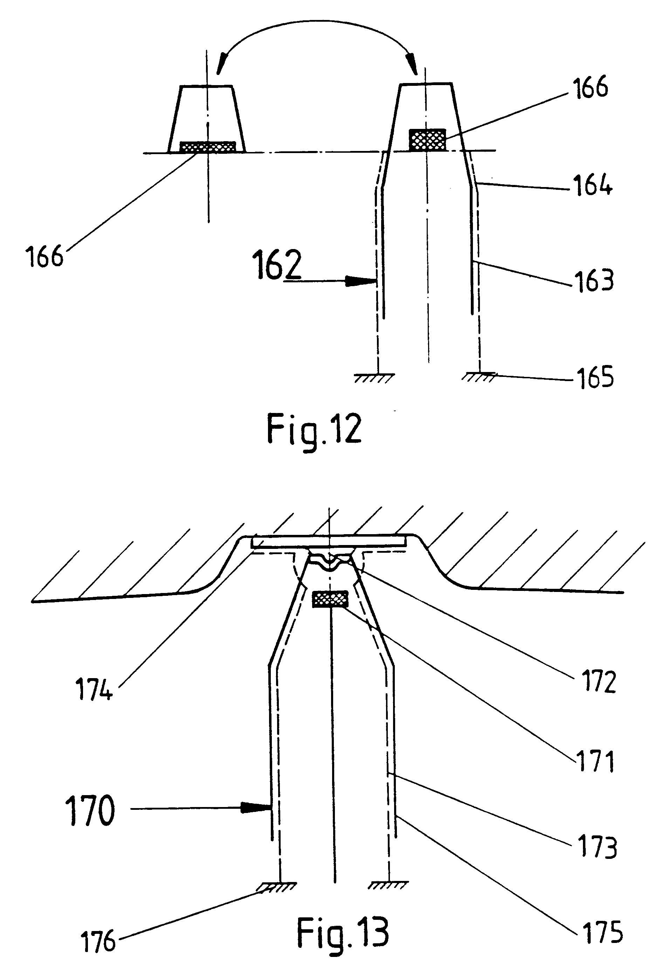

Fig. 11 zeigt eine andere Ausführungsform dieser Abtastvorrichtung 155 mit einer in einem Gehäuse 157 angeordneten Elektrode 159, welche Abtastvorrichtung 155 einen Metallring 156 als Anschlagkante beispielsweise an derjenigen Stelle ihres Gehäuses 157 aufweist, der etwa der Grenze der Metallisierung nach den Figuren 8 und 9 entspricht und der ebenfalls mit Masse 160 über eine wahlweise als Abschirmung dienende Ableitung 158 verbunden ist. Mit dieser Konstruktion erfolgt bei Verkanten (schrägem Ansetzen der Abtastvorrichtung auf der Haut-- hier abgebildet) oder zu tiefem Eindrücken auf dem Gewebe eine schnelle und sichere Ableitung der Hochfrequenz an Masse 160 und damit ein besonders deutlich sichtbarer Abfall des Zeigers an Meßinstrument (oder Veränderung der Zahlenangabe am Digitalinstrument) und damit ein sofortiger Hinweis an den Messenden, daß die Messung nicht korrekt ausgeführt wurde. Die in den Fig. 8, 9 und 11 beispielhaft dargestellte Möglichkeit der Ableitung der Hochfrequenz an Masse bietet die unerwartete günstige Möglichkeit der Übertragung gerade derjenigen gemessenen Daten an einen Datenspeicher und Datenverarbeiter (Computer, Bildschirm, Schreiber), die den jeweiligen höchsten Wert einer Messung darstellen, also unmittelbar vor dem Abfall des Zeigers. Auf diese Weise vereinfacht sich die Handhabung bei der Messung ganz entscheidend und bietet reproduzierbare Meßwerte, wenn die Vorrichtung vorher mit gleicher Handhabung auf einen Bezugspunkt des zu messenden Körpers geeicht worden ist.

Fig. 12 zeigt in einer weiteren Ausführungsform der Abtastvorrichtung 162 schematisch eine Elektrode 166 in einem auswechselbaren Tastkörperteil eines Gehäuses 163, welcher Tastkörperteil gegen andere Tastkörperteile mit Elektroden 166 verschiedener Form und/oder Größe auswechselbar eingerichtet ist, um von vornherein eine höhere oder geringere Empfindlichkeit (Aufnahmefähigkeit für die Feldstärke) zu erreichen. Auf Gem Gehäuse ist ein, Abschirmung 164 mit Masseanschluß 165 angeordnet, die bei Wechsel des Tastkörperteils unterschiedliche Wirkungen aufweist.

Fig. 13 zeigt eine weitere Ausführungsform der Abtastvorrichtung 170 mit einer in einem Gehäuse 175 angeordneten Elektrode 171, welche eine an der Spitze vorzugsweise mittels Druckknopfverbindung 172 (auch andere Haftverbindung ist möglich) angesetzte biegsame Scheibe 174 (oder anders geformte Fläche, wie Viereck), die in unterschiedlicher Größe auswechselbar zur Verfügung stehen kann, aufweist. Es ist eine von der Innenseite der biegsamen Scheibe 174 über die Außenseite des Gehäuses 175 und an der Innenseite des Gehäuses 175 weitergeführte Abschirmung 173 mit Masseanschluß 176 angeordnet. Mit dieser Konstruktion und einer entsprechenden Materialwahl (möglichst geringe Dielektrizitätskonstante des Materiales) für die Scheibe 174 können ebenfalls unerwünschte seitliche Einwirkungen, die durch verformte Oberflächenteile entstehen und damit eine fälschliche Erhöhung der empfangenen Feldstärke bewirken, vermieden werden. Die Scheibe 174 kann auch aus unterschiedlichem Material bestehen, das heißt aus verschiedenen Materialien mit jeweils unterschiedlicher Dielektrizitätskonstante zusammengesetzt sein, um die Abstrahlung der Hochfrequenz auf die Empfängerelektrode 171 zu kanalisieren. Die Hochfrequenzaufnahmefähigkeit läßt sich auch durch unterschiedlich dimensionierte und unterschiedlich angeordnete abschirmungen 173 regeln.

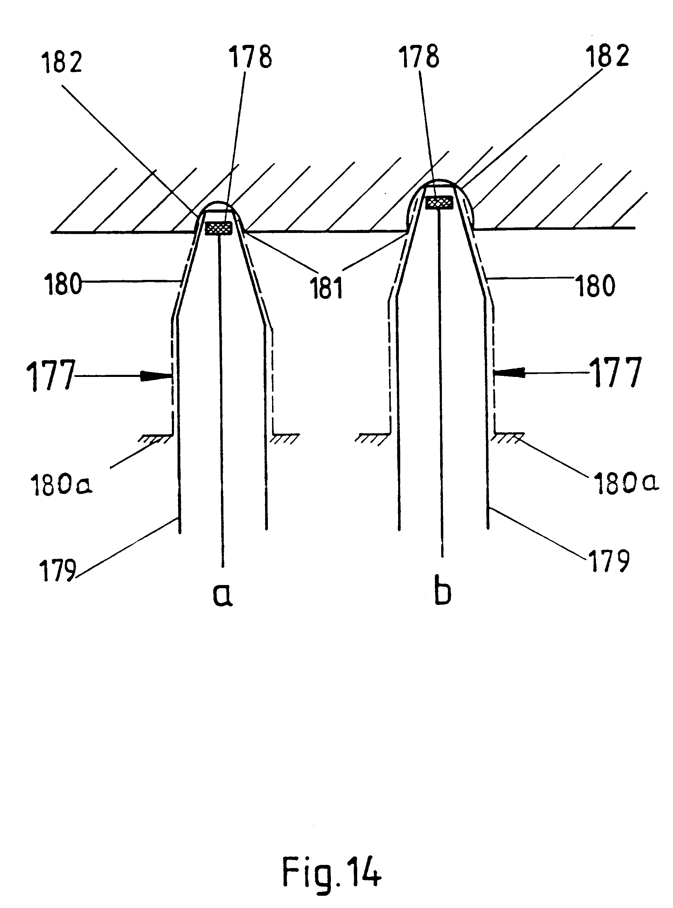

Fig. 14 zeigt als weitere Ausführungsform eine Abtastvorrichtung 177 mit einer Elektrode 178 in einem Gehäuse 179 und einer beispielsweise durch Silberleitlack bewirkten Abschirmung 180 am Gehäuse 179, wobei die metallische oder metallisierte Anschlagkante mit vorgegebener Berührungslinie 181 als spontan wirksame Ableitungsmöglichkeit der Hochfrequenz über die Abschirmung 180 zu Masse 180a dadurch veränderbar gemacht ist, daß die Spitze des Tastkörpers zur wahlweisen Aufnahme von isolierenden Kappen 182 unterschiedlicher Größe eingerichtet ist.

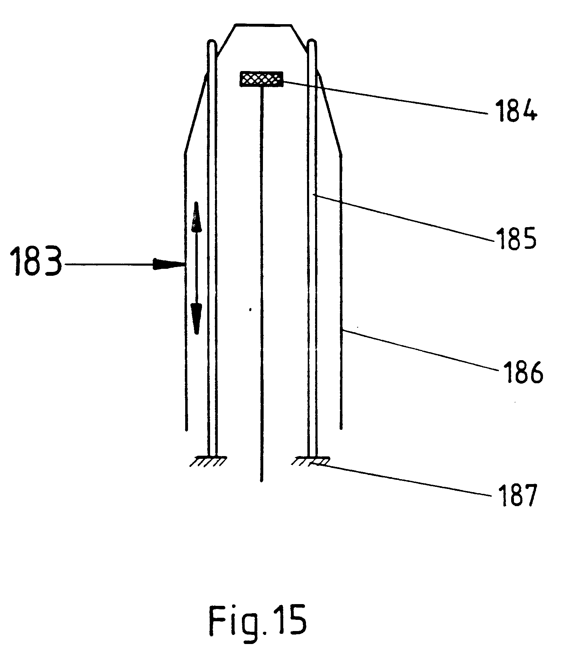

Fig. 15 zeigt die Ausführungsform einer Abtastvorrichtung 183 mit einer Elektrode 184 und einer Abschirmung 185 mit Masseanschluß 187 in einem Gehäuse 186, bei der die innen liegende Abschirmung 185 als vorne aus dem Gehäuse herausragendes und nach innen bewegbares Metallrohr 185 ausgeführt ist, das fest oder in seiner Längsachse verschiebbar angeordnet ist. Beim Herausragen des Metallrohres 185 kann die Hochfrequenz wie in Fig. 8, 9, 11, 12 und 14 spontan abgeleitet werden. Die Veränderung der Lage der Abschirmung 185 bewirkt eine Verminderung oder Erhöhung der Kapazität.

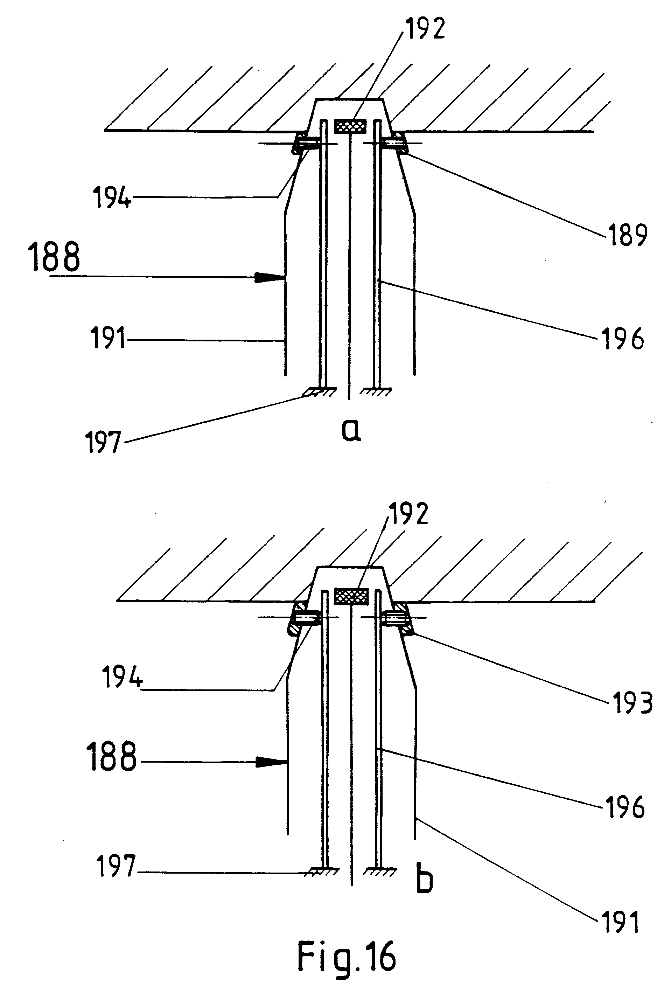

Fig. 16 zeigt eine in ihrem Gehäuse 191 eine Elektrode 192 aufweisende und an ihrem Gehäuse 191 mit einem Ring 189 mit Abschirmwirkung versehene Abtastvorrichtung 188, wobei der Ring 189 gegen einen anderen mit größerem oder kleinerem Rand 193 auswechselbar sein kann und mittels kontaktbewirkender Madenschrauben 194 an der die Weiterführung der durch den Ring 189, 193 gebildeten Abschirmung darstellenden innen liegenden Abschirmung 196 mit Masseanschluß 197 angeordnet ist. Statt der innen liegenden Abschirmung 196 kann eine auf dem Gehäuse 191 angeordnete Abschirmung vorliegen oder lediglich der abschirmend wirkende, als Anschlagkante dienende Ring 189, 193 an Masse angeschlossen sein. Die Wirkung der Berührung des Ringes 189, 193 mit der Haut entspricht der insbesondere in Fig. 11 und 15 beschriebenen.

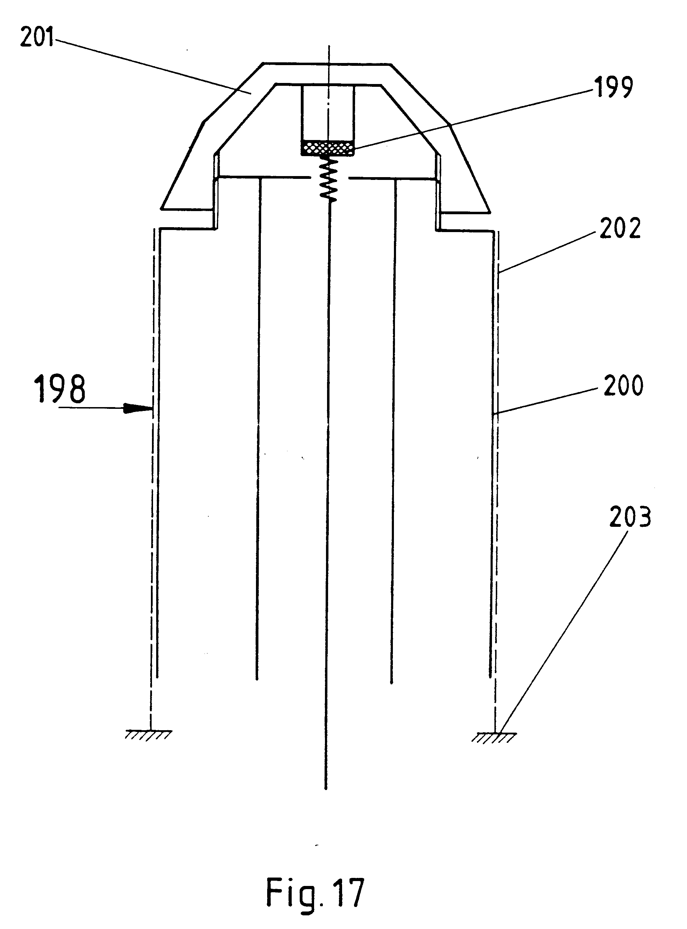

Fig. 17 zeigt eine Abtastvorrichtung 198 mit Elektrode 199 in einem Gehäuse 200, bei der der vordere Teil 201 des Gehäuses 200 verschiebbar oder verschraubbar angesetzt ist, um die Wirkung der auf dem Gehäuse 200 angeordneten Abschirmung 202 mit Masseanschluß 203 zu verändern.

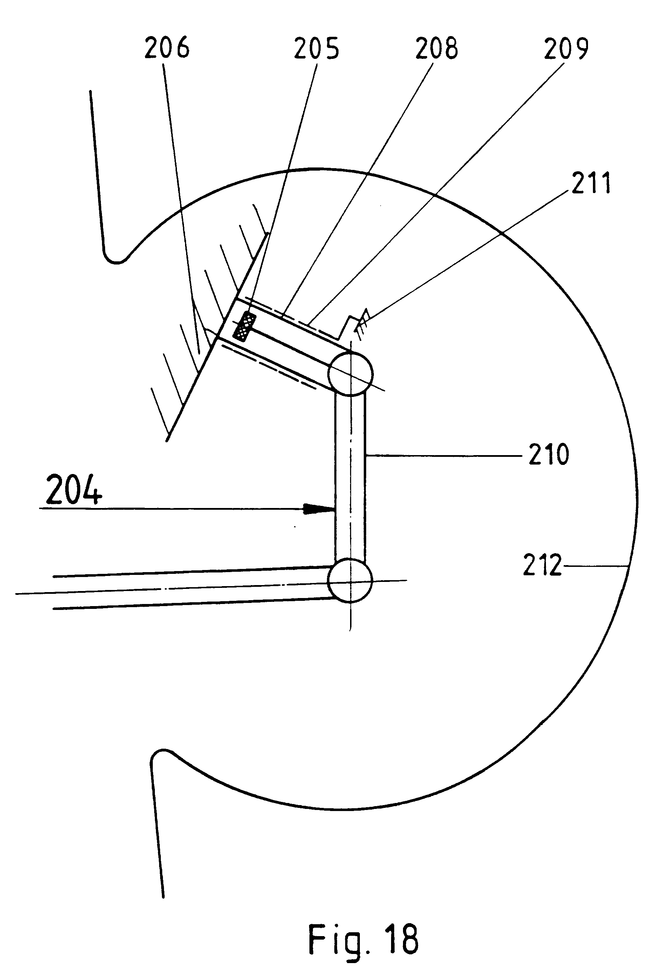

Fig. 18 zeigt eine Abtastvorrichtung 204 mit einer Elektrode 205 und einer Abschirmung 209 mit Masseanschluß 211 auf dem vorderen Teil 208 ihres Gehäuses die für die Testung am Zahnfleisch 206 innen (oder auch außen) oder an anderer Stelle im Rachengebiet 212 eingesetzt werden kann. In dieser ist der vordere Teil 208 und gegebenenfalls auch ein hinterer Teil 210 schwenkbar ausgeführt, um an schwer zugängliche Stellen des Rachens oder des Zahnfleisches zu gelangen und dennoch den vorderen Teil der Abtastvorrichtung senkrecht auf die zu testende Oberfläche aufsetzen zu können, weil trotz seitlicher Abschirmung 209 bei Kippen der Elektrode Meßfehler entstehen könnten.

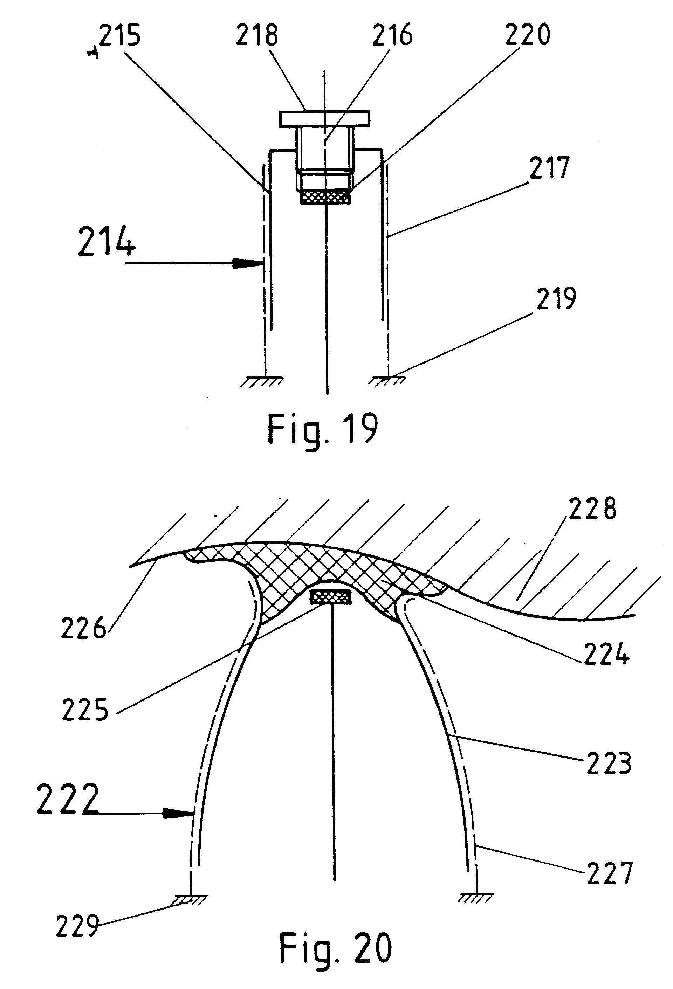

Fig. 19 zeigt eine Abtastvorrichtung 214 beispielsweise für den Rachenraum oder die Vagina mit einer in einem Gehäuse 215 angeordneten Elektrode 220 und einer am Gehäuse 215 vorne liegenden Verstellschraube 216, vorzugsweise aus Kunststoff, um den Abstand zwischen Aufsetzfläche 210 und Elektrode 220 durch Herein- bzw. Hinausschrauben zu verändern; die Wirkung der Abschirmung nimmt je nach Stellung der Verstellschraube 216 zu oder ab. Auf dem Gehäuse 215 ist eine Abschirmung 217 mit Masseanschluß 219 angeordnet.

Fig. 20 zeigt eine Abtastvorrichtung 222 mit einem Gehäuse 223, in welchem eine Elektrode 225 angeordnet und welches an ihrem vorderen Teil vor der Elektrode 225 mit einem angebauten oder abnehmbaren verformbaren Stück 224 versehbar ist, das sich an die Oberfläche 226 des zu messenden Körpers angleicht, damit nicht entweder Luft zwischen Meßfläche 226 und Abtastvorrichtung 222 tritt (Ergebnis: fälschliche Verminderung des Meßwertes) oder aber ein Eindrücken erfolgt, wodurch Gewebe 228 verdrückt und dadurch eine erhöhte Feldabstrahlung auch durch die Seitenwirkung des Gewebes 228 bewirkt wird (Ergebnis: fälschliche Erhöhung des Meßwertes). Auch dieses verformbare Stück kann wie die Scheibe nach Fig. 13 aus unterschiedlichen Materialien zusammengesetzt sein. An der Außenseite des Gehäuses 223 ist eine Abschirmung 227 mit Masseanschluß 229 angeordnet.

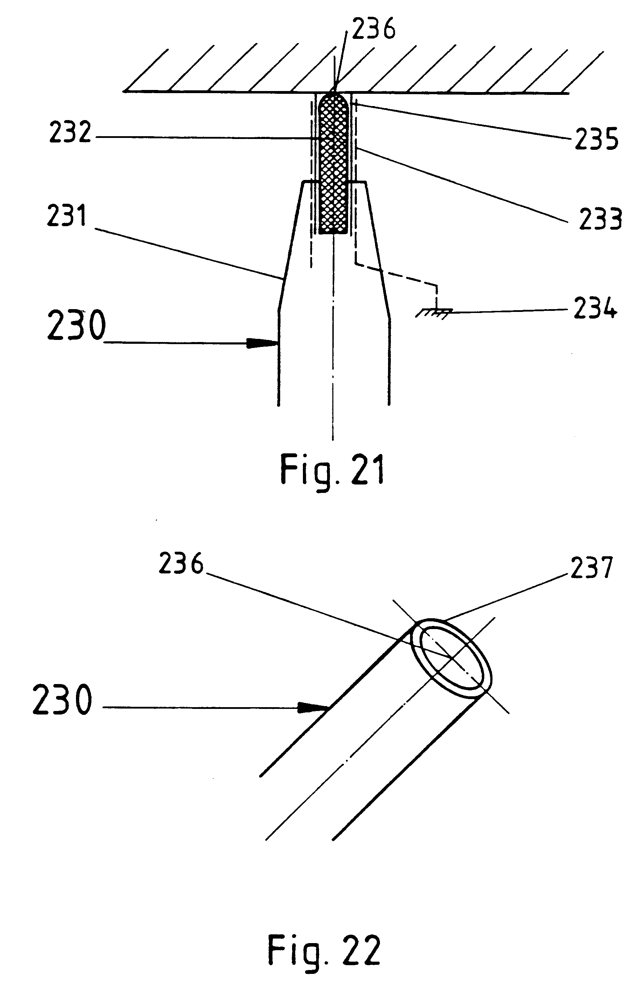

Fig. 21 zeigt eine schematische Darstellung einer Abtastvorrichtung 230 mit einer teilweise aus ihrem Gehäuse 231 nach vorne herausragenden, mit sehr geringem Abstand zur Meßoberfläche angeordneten Elektrode 232, die außen eine mit Masseanschluß 234 versehene Abschirmung 233 bei Zwischenschaltung einer Zwischenisolation 235 aufweist. Mit Hilfe dieser Abtastvorrichtung 230 können Akupunktur- oder Neuralpunkte des menschlichen oder tierischen Körpers einwandfrei und ohne Meßwertschwankungen aufgefunden werden. Der geringe Abstand zur Meßoberfläche kann beispielsweise durch einfachen Lackanstrich 236 erreicht werden. Ebenso kann eine Schutzlackierung über der Abschirmung 233 vorliegen.

Fig. 22 zeigt die Meßspitze 236 der in Fig. 21 dargestellten Abtastvorrichtung 230 mit deren runden Kante 237 (sie kann jedoch beliebig geformt sein). Mit diesem kann der gefundene Akupunktur- oder Neuralpunkt durch Druck auf die Haut für einige Sekunden markiert werden, um in dieser Zeit die Akupunkturnadel oder zum Beispiel ein handelsübliches aufklebbares Behandlungsmittel - auch mit eingeschlossener Nadelspitze - exakt an dem vorher gefundenen Punkt aufzusetzen, was die Wirkungsweise des Nadelstiches oder des Druckes eines nicht nadelspitzförmig geformten Materiales (beispielsweise von Metall, metallisiertem Kunststoff oder anderem akupunktur- oder neuralpunktwirksamem Material) optimiert.

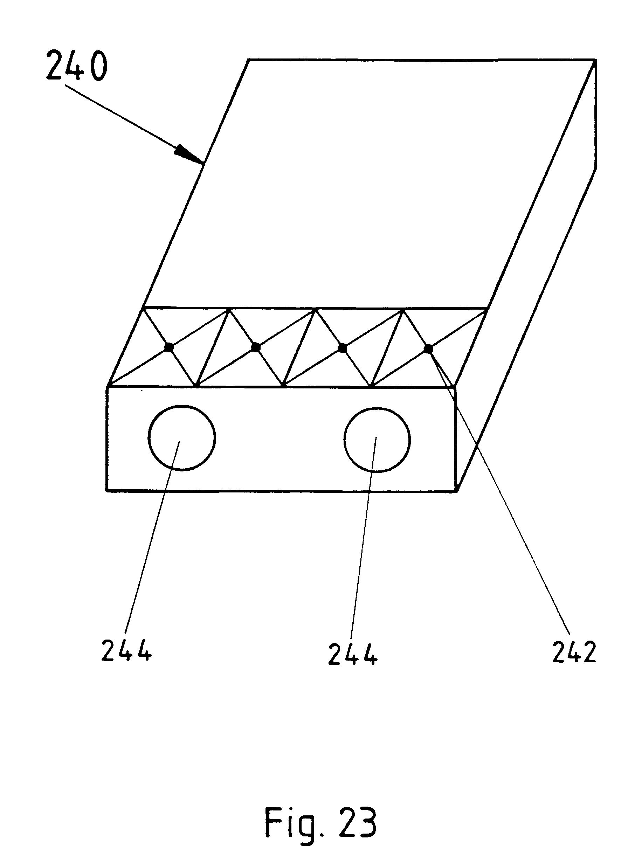

Fig. 23 zeigt eine metallene Sendeelektrode 240 in einer Ausführungsform beispielsweise für Tiermessungen, weil hier die Sendeelektrode 240 am Tier befestigt werden muß, beispielsweise mit einem Haftverschluß (wie Klettverschluß oder Riemen durch Löcher 244), wobei die Kontaktfläche der Sendeelektrode mit hervorstehenden Kanten oder Spitzen 242 oder anders geformten Erhebungen ausgestattet ist, um die Behaarung der Tierhaut, eventuell mit Hilfe von förderndem Kontaktgel, zu durchdringen und einen guten Leitkontakt herzustellen.

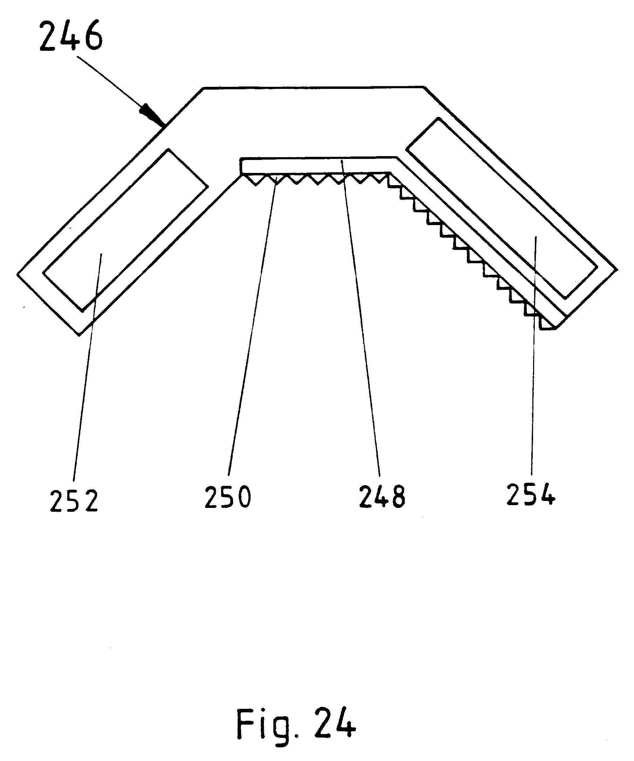

Fig. 24 zeigt eine Ausführungsform des Sendeteiles 246 einer Anlage mit Stromversorgung 252 und HF-Generator 254 schematisch, die beispielsweise bei Tiertestungen auf dem Rücken des Tieres durch einfaches Auflegen angebracht wird, wobei der eine metallene Teil 248, gegebenenfalls mit unterschiedlichen Oberflächenformungen 250 nach Fig. 23 versehen, den Kontakt mit der Tierkörperhaut herstellt; es kann der gesamte Sendeteil 246 der Anlage auch in einer Plastikhülle (hier nicht gezeichnet), auswechselbar oder fest, untergebracht sein. Die Stromversorgung 252 kann in dieser Ausführungsform auch entfallen, wenn die Stromversorgung von außen, beispielsweise vom Empfängerteil der Anlage her erfolgt.

In diese Fig. ist der Masseanschluß des Senderteiles 246, der mit dem Empfängerteil und zugleich mit der Abschirmung an der verwendeten Empfängerelektrode das gleiche Potential besitzt, nicht eingezeichnet

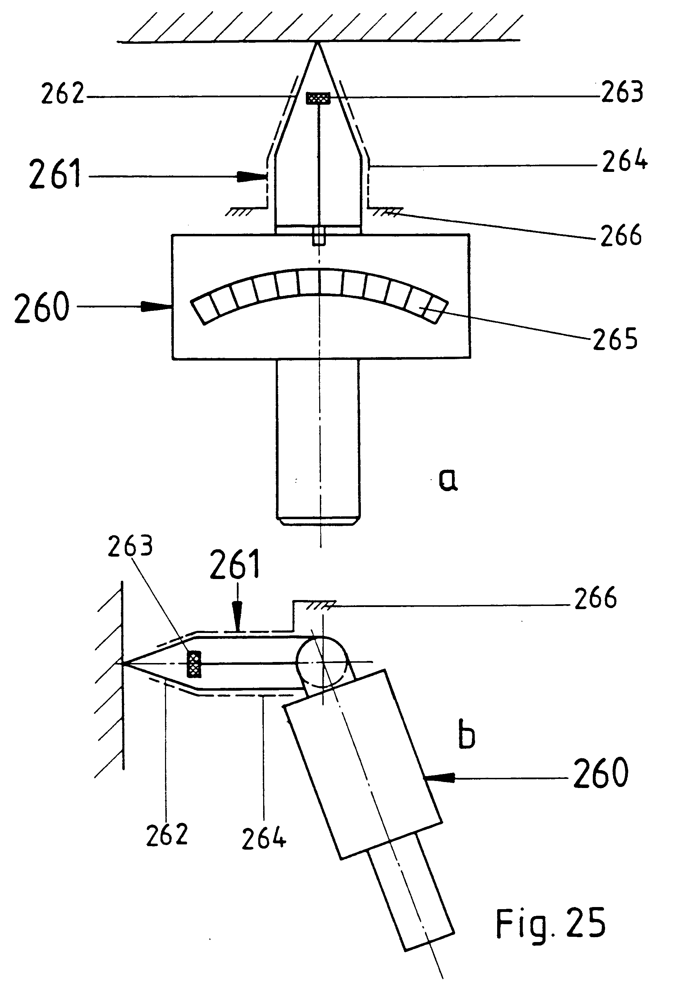

Fig. 25 zeigt eine Ausführungsform des Empfängerteiles 260 einer Anlage zusammen mit einem angesetzten Tastkörper 261 mit einem Gehäuse 262 mit einer Elektrode 263 und auf ihm angeordneter Abschirmung 264 mit Masseanschluß 266. Beispielsweise kann diese kombinierte Vorrichtung, weil sie ohne Verbindungskabel zwischen Empfängerteil 260 und Tastkörper 262 auskommt, gut für die Testung von Extremitäten, beispielsweise bei Pferden, eingesetzt werden, weil Kabel hindern. Es besteht die Möglichkeit, den Tastkörper 261 schwenkbar anzuordnen, um ein Ablesen des Analog- oder Digitalinstrumentes 265 zu erleichtern.
Wie bei Fig. 24 ist auch hier, wenn diese Ausführungsform nur den Empfängerteil 260 enthält, der gemeinsame Masseanschluß zum Sendeteil hin, der zugleich die Abschirmung der jeweils verwendeten Empfängerelektrode mit anschließt, nicht eingezeichnet. Die Stromversorgung für diesen Empfängerteil 260 kann innerhalb desselben, auch zugleich für den Senderteil der Anlage, angeordnet sein oder aber von außen durch eine gesonderte Stromversorgungsanlage erfolgen.

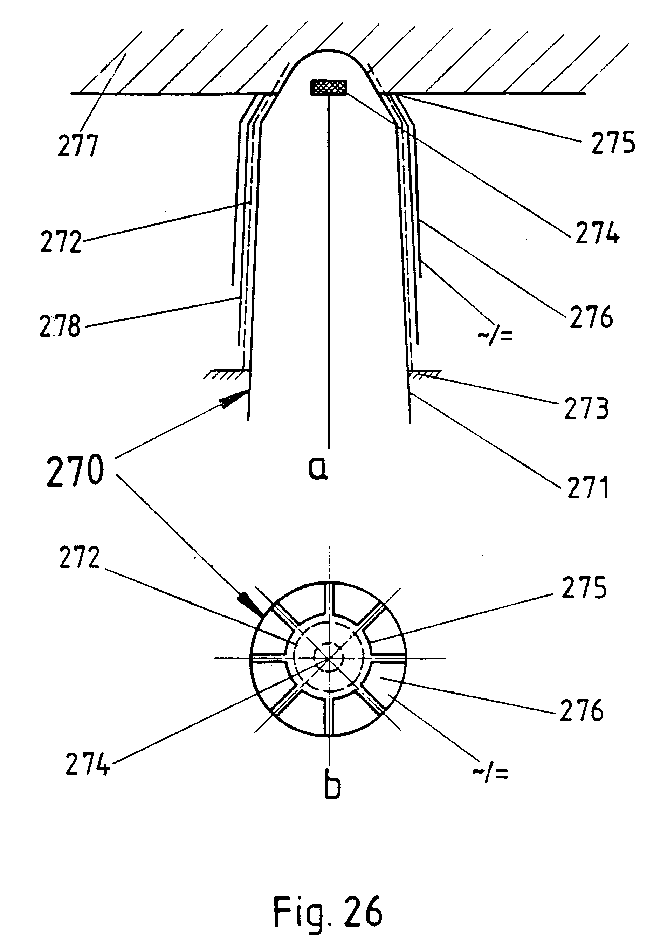

Fig. 26 zeigt eine Abtastvorrichtung 270 mit einer Elektrode 274 in einem Gehäuse 271, auf welchem eine an Masse 273 angeschlossene Abschirmung 272 und darüber, isoliert 278 gegen die Abschirmung, rundherum im Abstand voneinander an geringe, zweckmäßigerweise untereinander unterschiedliche Spannungen angelegte, und in eine bestimmte Reihenfolge gesetzte leitende Streifen 276 angeordnet sind. Die leitenden Streifen 276 haben jeweils eine Anschlagkante 275, welche bei entsprechendem Andruck des Gehäuses 271 den zu untersuchenden Körper 277 berührt, jedoch erst nach der Berührung der Anschlagkanten der Abschirmung 272. Die Berührung kann durch ein Registriergerät für alle leitenden Streifen 276 jeweils getrennt angezeigt werden. Die Abtastvorrichtung 270 hat nur dann die richtige senkrechte Ansatzstellung wenn die Anschlagkanten 275 von allen leitenden Streifen 276 den zu untersuchenden Körper 277 berühren und dementsprechend für alle leitenden Streifen 276 eine Anzeige, beispielsweise ein Aufleuchten von Lampen, vorliegt. Aus den etwaig fehlenden Anzeigen ergibt sich, in welcher Richtung fälschlich gekippt wurde.

Nach einer weiteren beispielhaften Ausführungsform sind auf einem Gehäuse, in welchem sich eine Abtastvorrichtung mit einer Elektrode befindet, außen rund herum in geringem Abstand voneinander an geringe, zweckmäßigerweise untereinander unterschiedliche, Spannungen angelegte leitende Streifen angeordnet. Die leitenden Streifen haben jeweils eine Anschlagkante, welche bei entsprechendem Andruck des Gehäuses den zu untersuchenden Körper berührt. Bei dieser Berührung wird die Hochfrequenz durch die leitenden Streifen abgeleitet (Funktion der ableitenden Anschlagkanten der Abschirmung, beispielsweise wie in Fig. 8, 9, 11, 12, 14, 15, 16 und 26, und zugleich wie bei der Beschreibung der Fig. 26 angegeben die Ansatzstellung der Abtastvorrichtung angezeigt: Gekipptsein (also falsch) oder senkrechte Stellung zur Körperoberfläche, (also richtig für die Messungen). Diese Ausführungsform kann auch wahlweise nur mit der einen Funktion (Abschirm- und Ableitfunktion) oder der anderen Funktion (Anzeige der Ansatzstellung der Abtastvorrichtung) oder mit beiden betrieben werden.

Nach einer noch weiteren beispielhaften Ausführungsform ist eine ähnliche Abtastvorrichtung wie die vorstehende mit einer in einem Gehäuse befindlichen Elektrode, jedoch mit vorne aus dem Gehäuse mit einem mehr oder weniger großen Teil herausragenden, nach innen bewegbaren, an Masse, oder geringe, zweckmäßigerweise untereinander unterschiedliche, Spannung angelegten leitenden Kontaktstreifen vorgesehen. Die Anschlagkanten dieser herausragenden vorderen Teile der Kontaktstreifen berühren bei entsprechendem Andruck des Tastkörpers die Oberfläche des zu messenden Körpers mit der gleichen Wirkung wie bei der vorstehend beschriebenen Ausführungsform angegeben: Funktion der Abschirmung und/oder Funktion der ableitenden Anschlagkanten mit daraus sich ergebender Funktion der Anzeige der Ansatzstellung der Abtastvorrichtung.

## Patentansprüche

1. Vorrichtung zum Feststellen von Eigenschaften, Verschiedenheiten und Veränderungen des menschlichen oder tierischen lebenden oder toten Körpers mit einer Wechselstromquelle, die mit einem Pol direkt oder indirekt an den Untersuchungskörper zur Bildung eines Hochfrequenzfeldes angeschlossen ist, und mit einer elektrischen Meßvorrichtung, die zwischen dem andeden Pol der Wechselstromquelle und einem über die Körperoberfläche geführten Tastkörper (177) mit einer Elektrode (178) angeordnet ist, wobei an der Elektrode (178) [ein] Abstandsstück(e) (182) vorgesehen ist/sind und die Elektrode (178) im Abstand zu der über den Körper geführten Fläche der Vorrichtung angeordnet ist, sowie die Elektrode (178) und/oder das Abstandsstück (182) und/oder das gegebenenfalls vorliegende Gehäuse (179)zur Abschirmung mit einer oder mehreren an Masse angeschlossenen Schicht(en) beziehungsweise Abdeckung(en) (180) aus leitfähigem Material oder Anstrichmaterial, von welchen bei Vorliegen eines Gehäuses (179) mindestens eine ganz oder teilweise außen auf dem Gehäuse (179) oder innerhalb dessen Wand angeordnet ist, versehen ist, dadurch gekennzeichnet, daß die, gegebenenfalls ganz oder teilweise mit einer Isolierung versehene Abschirmung (180) in Meßbetrieb mit dem zu messenden Körper eine Anschlagkante mit vorgegebener Berührungslinie (181) aufweist, über die zur Ableitung des durch die Vorrichtung erzeugten Hochfrequenzfeldes eine elektrische Verbindung mit Masse bzw. Erdpotential erfolgt, wobei die Berührungslinie (181) am Gehäuse (179) des Tastkörpers (177) dadurch veränderbar ist, daß der Tastkörper zur Aufnahme von Abstandstücken (182) verschiedener Größe eingerichtet ist, mit denen ein gewünschter Abstand zwischen der Elektrode (178) und den zu messenden Körper vorgegeben werden kann.

2. Vorrichtung nach Anspruch 1, dadurch gekennzeichnet, daß von der beziehungsweise den Abschirmung(en) (185) mindestens eine nach innen und außen bewegbar aus dem Gehäuse (186) herausragt.

3. Vorrichtung nach Anspruch 1 oder 2, dadurch gekennzeichnet, daß zwischen der Elektrode (86, 92, 139, 152, 159, 166, 171, 178, 184, 192, 205, 263, 274) und dem Ende des Gehäuses (82, 91, 138, 147, 157, 163, 175, 179, 186, 191, 208, 210, 262, 271) ein Freiraum vorgesehen ist.

4. Vorrichtung nach Anspruch 1 oder 2, dadurch gekennzeichnet, daß die Abstandsstücke (38, 224) aus verformbarem Material bestehen.

5. Vorrichtung nach einem oder mehreren der Ansprüche 1 bis 4, dadurch gekennzeichnet, daß das beziehungsweise die Abstandsstück(e) (38, 96, 98, 100, 174, 201, 216, 224), gegebenenfalls mit der Abschirmung (173, 227), lösbar auf dem Gehäuse (34, 91, 175, 200, 215, 223) angeordnet ist/sind.

6. Vorrichtung nach einem oder mehreren der Ansprüche 1 bis 5, dadurch gekennzeichnet, daß das beziehungsweise die Abstandsstück(e) (96, 98, 100, 112, 122) mit der Abschirmung (93, 113, 123) aus mehreren Materialien mit unterschiedlicher Dielektrizitätskonstante bestehen.

7. Vorrichtung nach einem oder mehreren der Ansprüche 1 bis 6, dadurch gekennzeichnet, daß zur wahlweisen Veränderung der Wirkung der Abschirmung (84) mehrere Abstandsstücke (96, 98, 100), gegebenenfalls austauschbar oder in der Zahl veränderbar, übereinander angeordnet sind.

8. Vorrichtung nach einem oder mehreren der Ansprüche 1 bis 7, dadurch gekennzeichnet, daß das mit der Abschirmung versehene Teil (84, 33, 93, 113, 123, 136, 154, 158, 164, 173, 180, 185, 189, 193, 196, 202, 209, 217, 227, 233, 264, 272, 282) des Gehäuses (82, 34, 91, 138, 147, 157, 163, 175, 179, 186, 191, 200, 208, 210, 215, 223, 231, 262, 271) in seiner Lage zu der Elektrode (86, 32, 92, 114, 124, 139, 152, 159, 166, 171, 178, 184, 192, 199, 205, 220, 225, 232, 263, 274) veränderbar ist.

9. Vorrichtung nach einem oder mehreren der Ansprüche 1 bis 8, dadurch gekennzeichnet, daß die Abschirmung (164) der Elektrode (166) lösbar zugleich mit der oder ohne die Elektrode (166) auf dem Gehäuse(163)angeordnet ist.

10. Vorrichtung nach einem oder mehreren der Ansprüche 1 bis 9, dadurch gekennzeichnet, daß die Abschirmung außen auf dem Gehäuse (138) der Elektrode (139) angebracht ist und daß die Stärke, die Dichte und/oder die Ausdehnung der Abschirmung (136) zur Gehäusespitze oder zur Elektrode (139) oder zum/zu den Abstandsstück(en) hin unterschiedlich ist/sind.

11. Vorrichtung nach einem oder mehreren der Ansprüche 1 bis 10, dadurch gekennzeichnet, daß das Material und/oder die Größe oder Form des/der Abstandsstücke[s] (36, 96, 98, 100, 216, 224) und/oder des umliegenden Gehäuseraumes und/oder -teiles so auf das Material oder die Größe oder Form der Elektrode (86, 32, 92, 139, 152, 159, 166, 171, 178, 184, 192, 199, 205, 220, 225, 232, 263, 274) abgestimmt ist beziehungsweise sind, daß die durch die Eindrückmulde der Meßkörperoberfläche bewirkte jeweilige Ausstrahlung der Feldstärke auf die Elektrode (86, 32, 92, 139, 152, 159, 166, 171, 178, 184, 192, 199, 205, 220, 225, 232, 263, 274) und/oder ihre Leitung (87) durch die Abschirmung kompensiert wird.

12. Vorrichtung nach Anspruch 10 oder 11, dadurch gekennzeichnet, daß die Stärke und das Material der Isolierung (89) der Abschirmung (84, 33, 93, 113, 123, 136, 154, 158, 164, 173, 180, 185, 189, 193, 196, 202, 209, 217, 227, 233, 264, 272), nach der gewünschten Ableitintensität der Anschlagkante (132, 156, 181, 189, 193 275) an Masse oder Erdpotential ausgeführt ist.

13. Vorrichtung nach einem oder mehreren der Ansprüche 1 bis 12, dadurch gekennzeichnet, daß außen am Gehäuse (157, 191) des Tastkörpers (155, 188) die Anschlagkante als mit einem an Masse (160, 197) oder an Erdpotential gelegter Ring (156, 190, 193) mit Abschirmwirkung, der fest oder in seiner Stellung veränderbar ist, ausgebildet ist.

14. Vorrichtung nach Anspruch 13, dadurch gekennzeichnet, daß der Ring (156, 189, 193) mit Abschirmwirkung entweder mit einer außen auf dem Gehäuse (157) des Tastkörpers (155) aufgebrachten Metallisierung oder über leitende Befestigungen oder Kontaktvorrichtungen mit der innerhalb des Gehäuses (191) des Tastkörpers (188) liegenden Abschirmung (196) verbunden ist.

15. Vorrichtung nach Anspruch 13 oder 14, dadurch gekennzeichnet, daß die als Ring (156, 189) ausgebildete Anschlagkante durch Auswechseln gegen einen solchen mit kleinerer oder größerer Außenfläche oder mit kleinerem oder größerem Rand (193) veränderbar ist.

16. Vorrichtung nach einem oder mehreren der Ansprüche 1 bis 15, dadurch gekennzeichnet, daß sie an ein Meßdatenregistriergerät (Computer) zur stetigen automatischen Erfassung der sich beim Meßvorgang in Funktion des Andruckes ergebenden Meßdaten mit abrufbarer Speicherung derjenigen beiden Höchstwerte oder eines der beiden, welche sich unmittelbar vor dem Beginn der Berührung und/oder unmittelbar nach der Beendigung der Berührung der Anschlagkanten (132, 181, 275, 156, 189, 193) mit Abschirmwirkung mit dem zu untersuchenden Körper einstellen, anschließbar ist.

17. Vorrichtung nach einem oder mehreren der Ansprüche 1 bis 16, dadurch gekennzeichnet, daß an der vorderen Seite oder Spitze des Gehäuses (175) des Tastkörpers (170) eine Druckknopfverbindung (172) oder eine sonstige lösbare Verbindung zur Befestigung einer, vorzugsweise biegsamen, Fläche (174), wahlweise verschiedener Größe und Form mit oder ohne Abschirmung (173) mit entsprechendem Verbindungsteil angeordnet ist.

18. Vorrichtung nach einem oder mehreren der Ansprüche 1 bis 17, dadurch gekennzeichnet, daß das Gehäuse (223) des Tastkörpers (222) einen vorderen fest oder abnehmbar angeordneten Teil (224) aus verformbarem, vorzugsweise biegsamem, Material mit oder ohne Abschirmung (227) aufweist, das sich der Körperoberfläche (226) angleicht.

19. Vorrichtung nach einem oder mehreren der Ansprüche 1 bis 18, dadurch gekennzeichnet, daß die innen in dem Gehäuse (186) der Elektrode (184) liegende metallene Abschirmung als festmontiertes oder in seiner Längsachse verschiebbares Rohr (185) ausgebildet ist, das zur Veränderung der Abschirmungswirkung unterschiedlich grobe, unterschiedlich auf die Rohrfläche (185) verteilte und unterschiedlich geformte Ausnehmungen besitzen kann.

20. Vorrichtung nach einem oder mehreren der Ansprüche 1 bis 19, dadurch gekennzeichnet, daß ein Vorderteil des Gehäuses (215) des Tastkörpers (214) aus einer ein- und ausdrehbaren Verstellschraube (216) zur Veränderung des Abstandes zwischen Elektrode (220) und Meßoberfläche (218) und zur Veränderung der Wirkung der Abschirmung (217) besteht.

21. Vorrichtung nach Anspruch 20, dadurch gekennzeichnet, daß die Verstellschraube (216) einen verformbaren unteren Teil zum Füllen des sich beim Herausdrehen der Verstellschraube bildenden Zwischenraumes mit elastischem verformbarem Material aufweist, das sich beim Hineindrehen zusammendrückt.

22. Vorrichtung nach einem oder mehreren der Ansprüche 1 bis 21, dadurch gekennzeichnet, daß das Abstandsstück der Elektrode (232) allein aus einer Lackierung oder einer anderen dünnen Auflage besteht, welche mehr oder weniger ausgedehnt mit einer Abschirmung (233) versehen sein kann, die über die ebenfalls lackierte oder eine andere dünne isolierende Auflage (235) aufweisende Elektrode (232) sich erstrecken kann.

23. Vorrichtung nach Anspruch 22, dadurch gekennzeichnet, daß die isolierte Elektrode (232) an ihrer vorderen Spitze (236) eine beliebig geformte Kante (237) zum leichten Markieren einer gefundenen Meßstelle auf der Haut aufweist.

24. Vorrichtung nach einem oder mehreren der Ansprüche 1 bis 23, dadurch gekennzeichnet, daß das HF-Sendeteil und das HF-Empfängerteil in getrennten Gehäusen angeordnet sind und durch Verbindung der beiden Masseteile dasselbe Massepotential besitzen wie die Abschirmung (84, 33, 93, 113, 123, 136, 154, 158, 164, 173, 180, 185, 189, 193, 196, 202, 209, 217, 227, 233, 264, 272) an den Tastkörpern (80, 30, 90, 110, 120, 134, 145, 155, 162, 170, 177, 183, 189, 198, 204, 214, 222, 230, 261, 270).

25. Vorrichtung nach einem oder mehreren der Ansprüche 1 bis 24, dadurch gekennzeichnet, daß ihr Sendeteil eine von der Abschirmung (84, 33, 93, 113, 123, 136, 154, 158, 164, 173, 180, 185, 189, 193, 196, 202, 209, 217, 227, 233, 264, 272) unbeeinflußt gebliebene hochfrequente Schwingung einer Frequenz von 150 bin 950 kHz ausstrahlt.

26. Vorrichtung nach Anspruch 25, dadurch gekennzeichnet, daß die Frequenz 250 kHz bis 700 kHz beträgt.

27. Vorrichtung nach einem oder mehreren der Ansprüche 1 bis 26, dadurch gekennzeichnet, daß über der Abschirmung (272) isoliert und gegeneinander und/oder nach außen isoliert leitende Kontakte (276), welche an geringe elektrische Spannungen anschließbar sind, fest oder verschiebbar angeordnet sind.

28. Vorrichtung nach Anspruch 27, dadurch gekennzeichnet, daß die leitenden Kontakte (276) mit nach vorne zum zu messenden Körper gerichteten Anschlagkanten (275) zur Berührung mit dem zu untersuchenden Körper (277) eingerichtet sind.

29. Vorrichtung nach Anspruch 27 oder 28, dadurch gekennzeichnet, daß die leitenden Kontakte außen auf dem Gehäuse des Tastkörpers oder in seiner Wand liegend, selbst als, gegebenenfalls alleinige, Abschirmung eingerichtet sind.

30. Vorrichtung nach einem oder mehreren der Ansprüche 1 bis 29, dadurch gekennzeichnet, daß die Abschirmung (84, 33, 93, 113, 123, 136, 154, 158, 164, 173, 180, 185, 189, 193, 196, 202, 209, 217, 227, 233, 264, 272) abschaltbar ist.

## Claims

1. An apparatus for detecting properties, differences and changes of human or animal, living or dead bodies, comprising an a.c. source, which has one terminal that is directly or indirectly connected to the body to be examined in order to produce a radio-frequency field, and an electric measuring device, which is coupled between the other terminal of the a.c. source and a sensing body (177) with an electrode (178), which is moved over the surface of the body, wherein at least one spacer (182) is provided on the sensing electrode (178) and the sensing electrode (178) is spaced from that surface of the apparatus which is moved in contact with the body, the electrode (178) and/or the spacer (182) and/or any housing (179) for shielding which is provided with at least one grounded layer or covering (180) consisting of conductive material or conductive paint, and, if a housing (179) is provided, at least one of said layers or coverings is disposed entirely or in part on the outside of the housing (179) or within the wall of the housing, characterized in that the shield (180), which is optionally provided entirely or in part with an insulation, has a forward edge (181), which is adapted to contact the body to be examined and to effect a leakage of the radio-frequency field produced by the apparatus. The boundary (181) at the housing (179) of the sensing body (177) is adjustable in such a way that spacers (182) of different sizes can be integrated into the sensing body, so that a desired space between the electrode (178) and the body to be examined can be determined.

2. An apparatus according to claim 1, characterized in that the shield or at least one of the shields (185) protrudes from the housing (186) and is movable into the housing.

3. Apparatus according to claim 1 or 2, characterized in that a clearance is provided between the electrode (86, 52, 139, 152, 155, 166, 171, 178, 184, 192, 205, 263, 274) and the end of the housing (82, 91, 138, 147, 157, 163, 175, 179, 186, 191, 208, 210, 262, 271)

4. An apparatus according to claim 1 or 2, characterized in that the spacers (38, 224) consist of deformable material.

5. An apparatus according to any of claims 1 to 4, characterized in that the at least one spacer (38, 96, 98, 100, 174, 201, 216, 224) and optionally also the shield (173, 227) is detachably mounted on the housing (34, 91, 175, 200, 215, 223).

6. An apparatus according to any of claims 1 to 5, characterized in that the at least one spacer (96, 98, 100, 112, 122) and the shields (93, 113, 123) consist of a plurality of materials having different dielectric constants.

7. An apparatus according to any of claims 1 to 6, characterized in that a selective change of the effect of the shield (84) is permitted in that a plurality of spacers (96, 98, 100) are arranged one over the other and said spacers are optionally interchangeable or can be altered in number.

8. An apparatus according to any of claims 1 to 7, characterized in that the position of the shielding part (84, 33, 93, 113, 123, 136, 154, 158, 164, 173, 180, 185, 189, 193, 196, 202, 209, 217, 227, 233, 264, 272, 282) of the housing (82, 34, 91, 138, 147, 157, 163, 175, 179, 186, 191, 200, 208, 210, 215, 223, 231, 262, 271) relative to the electrode (86, 32, 92, 114, 124, 139, 152, 159, 166, 171, 178, 184, 192, 199, 205, 220, 225, 232, 263, 274) is variable.

9. An apparatus according to any of claims 1 to 8, characterized in that the shield (164) associated with the electrode (166) is mounted on the housing (163) to be detachable from the housing (163) together with or without the electrode (166).

10. An apparatus according to any of claims 1 to 9, characterized in that the shield is provided on the outside of the housing (138) of the electrode (139) and the thickness, the density and/or the extent of the shield (136) toward the tip of the housing or to the electrode (139) or to the at least one spacer are different.

11. An apparatus according to any of claims 1 to 10, characterized in that the material and/or the size or shape of the at least one spacer (38, 96, 98, 100, 216, 224) and/or the surrounding housing space and/or housing part are selected to match the material or the size or shape of the electrode (86, 32, 139, 152, 159, 166, 171, 178, 184, 192, 199, 205, 220, 225, 232, 263, 274) in such a manner that the effect of the radiation pattern of the field intensity which is due to the depression in the surface of the body being examined on the electrode (86, 32, 92, 139, 152, 159, 166, 171, 178, 184, 192, 199, 205, 220, 225, 232, 263, 274) and/or the electrode lead (87) will be compensated by the shield.

12. An apparatus according to claim 10 or 11, characterized in that the thickness and the material of the insulation (89) provided on the shield (84, 33, 93, 113, 123, 136, 154, 158, 164, 173, 180, 185, 189, 193, 196, 202, 209, 217, 227, 233, 264, 272) are selected in dependence on the leakage which is to be effected by the contact with the forward edge of the shield (132, 156, 181, 189, 193, 275).

13. An apparatus according to any of claims 1 to 12, characterized in that the housing (157, 191) of the sensing body (155, 188) is provided on the outside with a shielding ring (156, 190, 193), which is connected to ground (160, 197) or to a low electric potential which effects a shielding or leakage like a ground potential and said shielding ring is fixed or is adjustable in position.

14. An apparatus according to claim 13, characterized in that the shielding ring (156, 189, 193) is connected to a metal layer which is provided on the outside of the housing (157) of the sensing body (155) or is connected by conductive fixing means or contacts to the shield (196) which is disposed inside the housing (191) of the sensing body (188).

15. An apparatus according to claim 13 or 14, characterized in that the shielding ring (156, 189) is replaceable by another shielding ring which has a smaller or larger outside surface or a smaller or larger rim (193).

16. An apparatus according to any of claims 1 to 15, characterized in that it is adapted to be connected to an apparatus (computer) for a recording of measured data, which apparatus serves for a continual automatic acquisition of the data which are measured in dependence on the pressure applied and for a retrievable storage of those two peak values which have been obtained immediately before the contact and/or immediately after the contact between the edges (132, 181, 275, 156, 189, 193) or the shielding rings and the body to be examined.

17. An apparatus according to any claims 1 to 16, characterized in that the housing (175) of the sensing body (170) is provided on its forward side portion or at its tip with a snap fastener (172) or with other fastening means for a detachable connection to mating fastening means provided on a preferably flexible surface (174) which may selectively have different sizes or shapes and may or may not be provided with a shield (173).

18. An apparatus according to any of claims 1 to 17, characterized in that the housing (223) of the sensing body (222) comprises a permanently or detachably mounted portion (224) which is made of a deformable and preferably flexible material and may be or may not be provided with a shield (227) and is adapted to adapt itself to the surface (226) of the body to be examined.

19. An apparatus according to any of claims 1 to 18, characterized in that the metallic shield which is disposed in the interior of the housing (186) for the electrode (184) consists of a tube (185), which is fixedly mounted or is displaceable along its longitudinal axis and which may be provided with apertures, which differ in size and shape and are differently distributed over the surface of the tube (185) and serve to change the shielding action.

20. An apparatus according to any of claims 1 to 19, characterized in that a front portion of the housing (215) of the sensing body (214) consists of an adjusting screw (216) which can be screwed in and out to change the distance between the electrode (220) and the surface (218) to be measured and for a change of the effect of the shield (217).

21. An apparatus according to claim 20, characterized in that the adjusting screw (216) comprises a deformable lower portion, which ensures that the space which is formed during an unscrewing of the adjusting screw will be filled with an elastically deformable material, which will be compressed as the adjusting screw is screwed in.

22. An apparatus according to any of claims 1 to 21, characterized in that the spacer associated with the electrode (232) consists only of a coat of paint or another thin insulating covering, which may be provided with a more or less extensive shield (233), which may extend over the electrode (232), which is also painted or provided with another then insulating covering (232).

23. An apparatus according to claim 22, characterized in that the insulated electrode (232) is formed at its forward tip (236) with an edge (237), which may have any desired shape and serves to conveniently mark a measuring location which has been found on the skin.

24. An apparatus according to any of claims 1 to 23, characterized in that the radio-frequency transmitting section and the radio-frequency receiving section are accommodated in separate housings and the same ground potential as the shield (84, 33, 93, 113, 123, 136, 154, 158, 164, 173, 180, 185, 189, 193, 196, 202, 209, 217, 227, 233, 264, 272) provided on the sensing bodies (80, 30, 90, 110, 120, 134, 145, 155, 162, 170, 177, 183, 188, 198, 204, 214, 222, 230, 261, 270) is applied to both sections because the two grounded parts are interconnected.

25. An apparatus according to any of claims 1 to 24, characterized in that its transmitting section radiates a radio-frequency oscillation at a frequency between 150 and 950 kHz, which oscillation has not been affected by the shield (84, 33, 93, 113, 123, 136, 154, 158, 164, 173, 180, 185, 189, 193, 196, 202, 209, 217, 227, 233, 264, 272,

26. An apparatus according to claim 25, characterized in that the frequency is between 250 and 700 kHz.

27. An apparatus according to any of claims 1 to 26, characterized in that conductive contacts (276), which are fixed or displaceable, are provided over the shield (272) and are insulated from the shield and from each other and/or from the outside and are adapted to be connected to sources of low electric voltages.

28. An apparatus according to claim 27, characterized in that the conductive contacts (276) are adapted at one end (275) to contact the body (277) that is to be examined.

29. An apparatus according to claim 27 or 28, characterized in that the conductive contacts are provided on the outside surface of the housing of the sensing body or in the wall thereof and the contacts constitute also shielding means or the entire shield.

30. An apparatus according to any of claims 1 to 29, characterized in that the shield (84, 33, 93, 113, 123, 136, 154, 158, 164, 173, 180, 185, 189, 193, 196, 202, 209, 217, 227, 233, 264, 272) is adapted to be disconnected.

## Revendications

1. Dispositif pour déterminer des propriétés, des différences et des changements du corps humain ou animal, vivant ou mort, avec une source de courant alternatif, qui est connectée avec un pôle directement ou indirectement au corps à examiner, pour former un champ haute fréquence, et avec un dispositif de mesure électrique qui est placé entre l'autre pôle de la source de courant alternatif et un corps palpeur (177) avec une électrode (178) conduit à la surface du corps, où une (des) pièce(s) d'écartement (182) est/sont prévue(s) sur l'électrode et l'électrode (178) est placée à distance de la surface du dispositif qui est conduit sur le corps, ainsi que l'électrode (178) et/ou la pièce d'écartement (182) et/ou le carter (179) existant le cas échéant pour l'écran est/sont muni(s) d'une ou plusieurs couches branchées à la masse ou recouvrement(s) (180) consistant en un matériel ou en un matériel peint conductible, dans lesquels en cas de présence d'un carter (179) au moins une ou un de ceux-ci est disposé(e) entièrement ou en partie à l'extérieur du carter (179) ou à l'intérieur de sa paroi, caractérisé en ce que l'écran (180) qui est garni, le cas échéant, entièrement ou en partie d'une isolation, forme un bord de butée avec une ligne de contact (181) déterminée à l'avance avec le corps à mesurer lors des mesures, par lequel a lieu une connexion électrique avec la masse ou avec le potentiel de terre pour la dérivation du champ haute fréquence produite par le dispositif, où la ligne de contact (181) sur le carter (179) du corps palpeur (177) en est modifiable, de telle sorte que le corps palpeur est réalisé pour prendre des pièces d'écartement (182) de grandeurs différentes, avec lesquelles un espace souhaité, entre l'électrode (178) et le corps à mesurer, peut être prédéterminé.

2. Dispositif selon la revendication 1, caractérisé en ce que l'écran ou au moins un des écrans (185) fait saillie du carter (186) de façon mobile vers l'intérieur et l'extérieur.

3. Dispositif selon la revendication 1 ou 2, caractérisé en ce qu'entre l'électrode (86, 92, 139, 152, 159, 166, 171, 178, 184, 192, 205, 263, 274) et l'extrémité du carter, (82, 91, 138, 147, 157, 163, 175, 179, 186, 191, 208, 210, 262, 271) il est prévu un espace libre.

4. Dispositif selon la revendication 1 ou 2, caractérisé en ce que les pièces d'écartement (38, 224) sont composées de matériel déformable.

5. Dispositif selon une ou plusieurs des revendications, de 1 à 4, caractérisé en ce que la (les) pièce(s) d'écartement (38, 96, 98, 100, 174, 201, 216, 224) selon le cas avec l'écran (173, 227) est/sont disposée(s) sur le carter (34, 91, 175, 200, 215, 223) de façon détachable.

6. Dispositif selon une ou plusieurs des revendications de 1 à 5, caractérisé en ce que la (les) pièce(s) d'écartement ( 96, 98, 100, 112, 122) est/sont composée(s) avec l'écran (93, 113, 123) de plusieurs matériels ayant des constantes diélectriques différentes.

7. Dispositif selon une ou plusieurs des revendications de 1 à 6, caractérisé en ce que pour un changement sélectif de l'effet de l'écran (84), plusieurs pièces d'écartement (96, 98, 100) sont disposées l'une sur l'autre, en option de façon interchangeable ou de façon modifiable dans leur nombre.

8. Dispositif selon une ou plusieurs des revendications de 1 à 7, caractérisé en ce que la partie (84, 33, 93, 113, 123, 136, 154, 158, 164, 173, 180, 185, 189, 193, 196, 202, 209, 217, 227, 233, 264, 272, 282) du carter (82, 34, 91, 138, 147, 157, 163, 175, 179, 186, 191, 200, 208, 210, 215, 223, 231, 262, 271) ayant l'écran est modifiable dans sa position relative à l'électrode (86, 32, 92, 114, 124, 139, 152, 159, 166, 171, 178, 184, 192, 199, 205, 220, 225, 232, 263, 274 ).

9. Dispositif selon une ou plusieurs des revendications de 1 à 8, caractérisé en ce que l'écran (164) de l'électrode (166) est disposé dans le carter (163) ensemble avec ou sans i'électrode (166) pour être détachable.

10. Dispositif selon une ou plusieurs des revendications de 1 à 9, caractérisé en ce que l'écran est placé à l'extérieur du carter (138) de l'électrode (139) et que l'épaisseur, la densité et/ou la dimension de l'écran (136) est/sont différente(s) vers le haut du carter ou vers l'électrode (139) ou vers au moins une des pièces d'écartement.

11. Dispositif selon une ou plusieurs des revendications de 1 à 10, caractérisé en ce que le matériel et/ou la grandeur ou la forme de la (des) pièce(s) d'écartement (38, 96, 98, 100, 216, 224) et/ou de l'espace du carter et/ou de la partie du carter est ou sont adapté(s) au matériel ou à la grandeur ou à la forme de l'électrode (86, 32, 92, 139, 152, 159, 166, 171, 178, 184, 192, 199, 205, 220, 225, 232, 263, 274) de telle sorte que chaque rayonnement de l'intensité du champ sur l'électrode (86, 32, 92, 139, 152, 159, 166, 171, 178, 184, 192, 199, 205, 220, 225, 232, 263, 274) et/ou sur la conduite de l'électrode (87), causé par l'effet de dépression de la surface du corps, est compensé par l'écran.

12. Dispositif selon les revendications 10 ou 11, caractérisé en ce que l'épaisseur et le matériel de l'isolation (89) de l'écran (84, 33, 93, 113, 123, 136, 154, 158, 164, 173, 180, 185, 189, 193, 196, 202, 209, 217, 227, 233, 264, 272) sont sélectionnés d'après l'intensité de la dérivation souhaitée du bord de butée (132, 156, 181, 189, 193, 275) à la masse ou au potentiel de terre.

13. Dispositif selon une ou plusieurs des revendications, de 1 à 12, caractérisé en ce qu'à l'extérieur du carter (157, 191) du corps palpeur (155, 188), le bord de butée est réalisé comme un anneau (156, 190, 193) posé à la masse (160, 197) ou au potentiel de terre, ayant un effet d'écrannage, qui est fixe ou modifiable dans sa position.

14. Dispositif selon la revendication 13, caractérisé en ce que l'anneau (156, 189, 193) ayant un effet d'écrannage est connecté soit avec une métallisation placée à l'extérieur du carter (157) du corps palpeur (155) soit par des moyens de fixation conductibles ou des dispositifs de contact avec l'écran (196), posé à l'intérieur du carter (191) du corps palpeur (188).

15. Dispositif selon la revendication 13 ou 14, caractérisé en ce que le bord de butée réalisé comme un anneau (156, 189) est remplaçable par un autre qui a une surface extérieure plus petite ou plus grande ou un bord plus petit ou plus large (193).

16. Dispositif selon une ou plusieurs des revendications, de 1 à 15, caractérisé en ce qu'il est adapté pour être connecté à un appareil d'enregistrement de données mesurées (ordinateur), pour l'acquisition automatique continue de données qui sont mesurées lors du déroulement de mesures en fonction de la pression appliquée et avec une mémorisation récupérable de ces deux valeurs maximales ou d'une des deux qui a été obtenue immédiatement avant le début du contact et/ou immédiatement après la fin du contact des bords de butée (132, 181, 275, 156, 189, 193) avec un effet d'écrannage, avec le corps à examiner.

17. Dispositif selon une ou plusieurs des revendications, de 1 à 16, caractérisé en ce que le carter (175) du corps palpeur (170) est muni, sur la partie latérale avant ou sur la tête, d'un bouton à pression (172) ou d'une autre connexion détachable pour fixer une surface (174), de préférence flexible, de grandeur et de forme différentes sélectives avec ou sans écran (173) ayant un connecteur correspondant.

18. Dispositif selon une ou plusieurs des revendications, de 1 à 17, caractérisé en ce que le carter (223) du corps palpeur (222) présente une partie frontale fixe ou détachable (224) qui est faite à partir d'un matériel déformable, de préférence flexible, avec ou sans écran (227) et qui s'adapte elle-même à la surface du corps (226).

19. Dispositif selon une ou plusieurs des revendications, de 1 à 18, caractérisé en ce que l'écran métallique qui se trouve à l'intérieur du carter (186) de l'électrode (184) est formé d'un tube (185), fixe ou coulissant le long de son axe longitudinale, qui peut posséder des creux de grandeur et de forme différentes et répartis différemment sur la surface du tube (185), pour modifier l'effet de l'écrannage.

20. Dispositif selon une ou plusieurs des revendications, de 1 à 19, caractérisé en ce qu'une partie frontale du carter (215) du corps palpeur (214) consiste en une vis de réglage (216), vissable ou dévissable pour modifier la distance entre l'électrode (220) et la surface (218) à mesurer et pour modifier l'effet de l'écrannage (217).

21. Dispositif selon la revendication 20, caractérisé en ce que la vis de réglage (216) présente une partie inférieure déformable pour remplir l'espace intermédiaire formé en dévissant la vis de réglage avec un matériel déformable élastique, qui s'écrase en vissant la vis.

22. Dispositif selon une ou plusieurs des revendications, de 1 à 21, caractérisé en ce que la pièce d'écartement de l'électrode (232) consiste seulement en une laque ou une autre couche d'isolation fine qui peut être munie d'un écran (233) plus ou moins extensible, qui peut s'étendre sur l'électrode (232) qui est laquée également ou munie d'une autre couche isolante fine (235).

23. Dispositif selon la revendication 22, caractérisé en ce que l'électrode isolante (232) a sur sa tête frontale (236) un bord (237) qui petit avoir une forme quelconque pour marquer légèrement une position de mesure trouvée sur la peau.

24. Dispositif selon une ou plusieurs des revendications, de 1 à 23, caractérisé en ce que la partie de transmission haute fréquence et la partie de réception haute fréquence sont disposées dans des carters séparés et qu'elles possèdent, par connexion avec les deux parties de masse, le même potentiel de masse que l'écran (84, 33, 93, 113, 123, 136, 154, 158, 164, 173, 180, 185, 189, 193, 196, 202, 209, 217, 227, 233, 264, 272) sur le corps palpeur (80, 30, 90, 110, 120, 134, 145, 155, 162, 170, 177, 183, 188, 198, 204, 214, 222, 230, 261, 270).

25. Dispositif selon une ou plusieurs des revendications, de 1 à 24, caractérisé en ce que sa partie de transmission émet une oscillation haute fréquence d'une fréquence entre 150 et 950 kHz, qui n'a pas été affectée par l'écran (84, 33, 93, 113, 123, 136, 154, 158, 164, 173, 180, 185, 189, 193, 196, 202, 209, 217, 227, 233, 264, 272).

26. Dispositif selon la revendication 25, caractérisé en ce que la fréquence se situe entre 250 kHz et 700 kHz.

27. Dispositif selon une ou plusieurs des revendications, de 1 à 26, caractérisé en ce que des contacts conductibles (276) sont disposés, fixes ou mobiles, et isolés de l'écran (272) et vis-à-vis l'un de l'autre et/ou isolés de l'extérieur et sont connectables à des sources de basse tension électrique.

28. Dispositif selon la revendication 27, caractérisé en ce que les contacts conductibles (276) sont disposés avec des bords de butée (275, orientés vers l'avant vers le corps à mesurer pour contacter le corps à examiner (277).

29. Dispositif selon les revendications 27 ou 28, caractérisé en ce que les contacts conductibles se trouvant à la surface extérieure du carter du corps palpeur ou dans sa paroi, sont disposés eux-mêmes comme écran ou le cas échéant comme écran seul.

30. Dispositif selon une ou plusieurs des revendications, de 1 à 29, caractérisé en ce que l'écran (84, 33, 93, 113, 123, 136, 154, 158, 164, 173, 180, 185, 189, 193, 196, 202, 209, 217, 227, 233, 264, 272) est déconnectable.
